(19) Europäisches Patentamt / European Patent Office / Office européen des brevets

(11) **EP 1 646 603 B1**

(12) **EUROPÄISCHE PATENTSCHRIFT**

(45) Veröffentlichungstag und Bekanntmachung des Hinweises auf die Patenterteilung:
**23.09.2009   Patentblatt 2009/39**

(21) Anmeldenummer: **04740862.0**

(22) Anmeldetag: **09.07.2004**

(51) Int Cl.:
*C07C 51/43* (2006.01)      *B01D 3/14* (2006.01)

(86) Internationale Anmeldenummer:
**PCT/EP2004/007583**

(87) Internationale Veröffentlichungsnummer:
**WO 2005/007610 (27.01.2005 Gazette 2005/04)**

(54) **THERMISCHES TRENNVERFAHREN ZUR ABTRENNUNG WENIGSTENS EINES (METH) ACRYLMONOMERE ANGEREICHERT ENTHALTENDEN STOFFSTROMS**

THERMAL SEPARATING METHOD FOR SEPARATING AT LEAST ONE MASS FLUX CONTAINING ENRICHED (METH)ACRYLIC MONOMERS

PROCEDE DE SEPARATION THERMIQUE POUR SEPARER AU MOINS UN FLUX DE MATIERE ENRICHIE EN MONOMERES (METH)ACRYLIQUES

(84) Benannte Vertragsstaaten:
**AT BE BG CH CY CZ DE DK EE ES FI FR GB GR HU IE IT LI LU MC NL PL PT RO SE SI SK TR**

(30) Priorität: **11.07.2003   DE 10331720**
**11.07.2003   US 486168 P**
**17.07.2003   DE 10332758**
**17.07.2003   US 487587 P**

(43) Veröffentlichungstag der Anmeldung:
**19.04.2006   Patentblatt 2006/16**

(73) Patentinhaber: **BASF SE**
**67056 Ludwigshafen (DE)**

(72) Erfinder:
• **ECK, Bernd**
**68519 Viernheim (DE)**

• **HAMMON, Ulrich**
**68163 Mannheim (DE)**
• **MÜLLER-ENGEL, Klaus, Joachim**
**76297 Stutensee (DE)**
• **SCHRÖDER, Jürgen**
**67071 Ludwigshafen (DE)**
• **THIEL, Joachim**
**67435 Neustadt (DE)**
• **MARTAN, Hans**
**67227 Frankenthal (DE)**

(56) Entgegenhaltungen:
**EP-A- 0 779 268           EP-A- 1 110 940**
**DE-A- 10 256 147           US-A1- 2003 018 214**

**Beschreibung**

Vorliegende Erfindung betrifft ein thermisches Trennverfahren zur Abtrennung wenigstens eines (Meth)acrylmonomere angereichert enthaltenden Stoffstroms aus einem (Meth)acrylmonomere enthaltenden Gemisch, umfassend den kontinuierlichen stationären Betrieb wenigstens einer thermischen Trennvorrichtung, die wenigstens einen trenn-wirksamen Raum mit oder ohne trennwirksame Einbauten umfasst, in den wenigstens ein (Meth)acrylmonomere enthaltender Stoffstrom hinein - und aus dem wenigstens ein (Meth)acrylmonomere enthaltender Stoffstrom herausgeführt wird, mit der Maßgabe, dass

-    der Stoffstrom, der insgesamt in den trennwirksamen Raum hineingeführt und gedanklich dadurch erzeugt wird, dass man die in den trennwirksamen Raum hineingeführten Einzelstoffströme addiert, X Gew.-% an von (Meth) acrylmonomeren verschiedenen Bestandteilen enthält,

-    der Stoffstrom, der mit dem höchsten Gewichtsanteil an (Meth)acrylmonomeren aus dem trennwirksamen Raum herausgeführt wird, Y Gew.-% an von (Meth)acrylmonomeren verschiedenen Bestandteilen enthält,

-    das Verhältnis X : Y ≥5 beträgt,

-    der trennwirksamen Raum, ausgenommen an den Stoffstromzufuhr- und an den Stoffstromausfuhrstellen von einer festen Phase begrenzt wird und wenigstens einen Umlaufwärmetauscher umfasst
und

-    das im trennwirksamen Raum mit Flüssigphase gefüllte Gesamtvolumen ≥1 m$^3$ beträgt, wobei die Temperatur der Flüssigphase wenigstens teilweise ≥80˚C beträgt.

Der Begriff stationärer Betrieb bedeutet in dieser Schrift, dass die Stoffströme in Bezug auf ihre Stoffgehalte und Strömungsmengen während des kontinuierlichen Betriebs in der jeweils gewählten Einheit um höchstens 5 %-Punkte variieren (bezogen auf den Mittelwert als Bezugsbasis). Erfindungsgemäß bevorzugt beträgt vorgenannte Varianz ≤4 % Punkte, besonders bevorzugt ≤3 % Punkte und ganz besonders bevorzugt ≤2 % Punkte oder ≤1 % Punkt.
Die Schreibweise (Meth)acrylmonomere steht in dieser Schrift verkürzend für "Acrylmonomere und/oder Methacrylmonomere".
Der Begriff Acrylmonomere steht in dieser Schrift verkürzend für "Acrolein, Acrylsäure und/oder Ester der Acrylsäure."
Der Begriff Methacrylmonomere steht in dieser Schrift verkürzend für "Methacrolein, Methacrylsäure und/oder Ester der Methacrylsäure".
Im besonderen sollen die in dieser Schrift angesprochenen (Meth)acrylmonomere die nachfolgenden (Meth) acrylsäureester umfassen: Hydroxyethylacrylat, Hydroxyethylmethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, iso-Butylmethacrylat, n-Butylmethacrylat, tert.-Butylacrylat, tert.-Btuylmethacrylat, Ethylacrylat, Ethylmethacrylat, 2-Ethylhexylacrylat, 2-Ethylhexylmethacrylat, N,N-Dimethylaminoethylmethacrylat und N,N-Dimethylaminoethylacrylat.
(Meth)acrylmonomere sind wichtige Ausgangsverbindungen zur Herstellung von Polymerisaten, die z.B. als Klebstoff Verwendung finden.
(Meth)acrolein und (Meth)acrylsäure wird großtechnisch überwiegend durch katalytische Gasphasenoxidation geeigneter C$_3$-/C$_4$-Vorläuferverbindungen (oder von Vorläuferverbindungen derselben), insbesondere von Propen und Propan im Fall von Acrolein und Acrylsäure bzw. iso-Buten und iso-Butan im Fall der Methacrylsäure und des Methacroleins, hergestellt.
Neben Propen, Propan, iso-Buten und iso-Butan eignen sich als Ausgangsstoffe jedoch auch andere 3 bzw. 4 Kohlenstoffatome enthaltende Verbindungen wie iso-Butanol, n-Propanol oder Vorläuferverbindungen derselben wie z.B. der Methylether von iso-Butanol. (Meth)acrylsäure kann auch aus (Meth)acrolein erzeugt werden.
Dabei wird normalerweise ein Produktgasgemisch erhalten, aus dem die (Meth)acrylsäure bzw. das (Meth) acrolein abgetrennt werden muß.
Ester der (Meth)acrylsäure sind z.B. durch direkte Umsetzung von (Meth)acrylsäure und/oder (Meth)acrolein mit den entsprechenden Alkoholen erhältlich. Allerdings fallen auch in diesem Fall zunächst Produktgemische an, aus denen die (Meth)acrylsäureester abgetrennt werden müssen.
Für vorstehende Abtrennungen werden häufig ein oder mehrere thermische Trennverfahren angewendet, wie es eingangs beschrieben ist. Diese werden üblicherweise kontinuierlich durchgeführt, wobei dem trennwirksamen Raum unter stationären Bedingungen kontinuierlich Stoffströme zugeführt und entnommen werden. Charakteristisch für thermische Trennverfahren ist, dass die mit ihnen erzielte Trennwirkung die Zufuhr (z.B. zum Verdampfen) und/oder den Entzug (z.B. zum Kondensieren) von thermischer Energie erfordert und dass an ihnen flüssige Phasen beteiligt sind (im trennwirksamen Raum geführt werden).

In der Regel wird diese thermische Energie über Umlaufwärmetauscher entzogen und/oder zugeführt. Ein Umlaufwärmetauscher ist ein Teil das beim eingangs beschriebenen Verfahren geforderten trennwirksamen Raums, dem aus einem anderen Teil des trennwirksamen Raums entnommene Flüssig- und/oder Gasphase zugeführt wird. Im Umlaufwärmetauscher wird dieser Flüssig- und/oder Gasphase von einer nicht vom trenn-wirksamen Raum umfassten Quelle auf direkte (z.B. dadurch, dass die Quelle in den Raum geführt wird) und/oder indirekte Weise thermische Energie zugeführt und/oder entzogen. Anschließend wird die abgekühlte oder erwärmte Flüssig (kann beim Wärmetausch teilweise und/oder vollständig in die Dampfphase überführt werden)- und/oder Gasphase (kann beim Wärmetausch teilweise und/oder vollständig kondensiert werden) vollständig in den anderen Teil des trennwirksamen Raums rückgeführt, wobei die Entnahme- und die Rückführstelle räumlich auseinander liegen können. In vielen Fällen ist der wenigstens eine Umlaufwärmetauscher ein Umlaufverdampfer.

Die Erzielung der thermischen Trennwirkung selbst (d.h., die Durchführung des thermischen Trennverfahrens) ist prinzipiell in trennwirksamen Räumen möglich, die keine trennwirksamen Einbauten umfassen, wie es z.B. bei einer einfachen Destillation der Fall ist. Dabei wird ein flüssiges Gemisch partiell verdampft und die dabei erzeugte, eine andere Zusammensetzung als das flüssige Gemisch aufweisende Dampfphase in dampfförmiger und/oder kondensierter Form abgetrennt.

Häufig wird die thermische Trennwirkung jedoch unter Mitwirkung trennwirksamer Einbauten erzielt, wobei vielfach gasförmige (meist aufsteigend) und flüssige (meist absteigend) Stoffströme im Gleich- oder Gegenstrom geführt werden. Infolge des zwischen den Stoffströmen bestehenden Ungleichgewichts findet ein Wärme- und Stoffaustausch statt, der letztlich die gewünschte Auftrennung bedingt. In der Regel befinden sich die trennwirksamen Einbauten in einer Trennkolonne.

Charakteristisch für die thermischen Trennverfahren dieser Schrift ist ferner, dass die durch den trennwirksamen Raum geführten individuellen chemischen Verbindungen sich beim Durchgang durch den trennwirksamen Raum zu weniger als 20-mol-% (jeweils bezogen auf die insgesamt in den trennwirksamen Raum geführte Gesamtmenge der jeweiligen individuellen chemischen Verbindung) chemisch verändern (ausgenommen die Michael-Addition der (Meth) acrylmonomeren; sie soll nicht als eine solche chemische Veränderung gewertet werden).

Häufig liegt der vorgenannte Prozentsatz bei den thermischen Trennverfahren dieser Schrift bei Werten ≤10 mol-%, oder ≤7 mol%, oder ≤3 mol-% bzw. ≤1 mol-%.

Beispiele für und damit Element des in dieser Schrift verwendeten Begriffs "thermisches Trennverfahren" sind die fraktionierende Kondensation (vgl. z.B. DE-A 19 924 532) und/oder die Rektifikation (aufsteigende Dampfphase wird im Gegenstrom zu absteigender Flüssigphase geführt; die Trennwirkung beruht darauf, dass die Dampfzusammensetzung im Gleichgewicht anders als die Flüssigzusammensetzung ist), die Absorption (wenigstens ein aufsteigendes Gas wird zu wenigstens einer absteigenden Flüssigkeit im Gegenstrom geführt; die Trennwirkung beruht auf der unterschiedlichen Löslichkeit der Gasbestandteile in der Flüssigkeit), die Strippung (wie die Absorption; die Flüssigphase ist jedoch mit einer Komponente beladen, die vom Strippgas aufgenommen wird) und die Desorption (der Umkehrprozess zur Absorption; das in der Flüssigphase gelöste Gas wird durch Partialdruckerniedrigung abgetrennt). Aber auch die Flüssig/Flüssig-Extraktion und die Kristallisation (insbesondere die Fallfilm- und die Suspensionskristallisation) sollen in dieser Schrift thermisches Trennverfahren sein.

Beispielsweise kann die Abtrennung von (Meth)acrylsäure bzw. (Meth)acrolein aus dem Produktgasgemisch der katalytischen Gasphasenoxidation von Propan und/oder Propen so durchgeführt werden, dass die (Meth)acrylsäure bzw. das (Meth)acrolein durch Absorption in ein Lösungsmittel (z.B. Wasser oder ein organischer Lösungsmittel) oder durch fraktionierende Kondensation des Produktgasgemisches zunächst grundabgetrennt und das dabei anfallende Kondensat bzw. Absorbat nachfolgend rektifikativ (in der Regel in mehreren Stufen) unter Erhalt von mehr oder weniger reiner (Meth)acrylsäure bzw. (Meth)acrolein aufgetrennt wird (vgl. z.B. EP-A 717 019, EP-A 1125 912, EP-A 982 289, EP-A 982287, DE-A 19 606 877, DE-A 1 011 527, DE-A 10 224 341, DE-A 10 218 419, DE-A 10 247 240 und DE-A 10 243 625).

Die vorstehend angesprochene fraktionierende Kondensation unterscheidet sich von der herkömmlichen Rektifikation im wesentlichen dadurch, dass das aufzutrennende Gemisch dem trennwirksamen Raum gasförmig (d.h., vollständig in die Dampfform überführt) zugeführt wird.

Die bereits angesprochenen, (Meth)acrylmonomere enthaltenden, gasförmigen und/oder flüssigen Gemische können die (Meth)acrylmonomere sowohl in mehr oder weniger reiner Form als auch in Verdünnung (z.B. mit Lösungsmittel oder mit Verdünnungsgasen) befindlich enthalten. Das Lösungsmittel kann dabei sowohl wässrig als auch ein organisches Lösungsmittel sein, wobei die spezifische Art des organischen Lösungsmittels im wesentlichen unbeachtlich ist. Das Verdünnungsgas kann z.B. Stickstoff, Kohlenoxid (CO, $CO_2$), Sauerstoff, Kohlenwasserstoff oder ein Gemisch aus diesen Gasen sein.

Das heißt, z.B. auf dem Weg der Gewinnung (Abtrennung) von (Meth)acrylmonomeren werden auf unterschiedlichste Art und Weise thermische Trennverfahren (wie sie eingangs beschrieben wurden) auf gasförmige und/oder flüssige Stoffgemische angewendet, deren Gehalt an (Meth)acrylmonomeren ≥2 Gew.-%, oder ≥10 Gew.-%, oder ≥20 Gew.-% oder ≥40 Gew.-%, oder ≥60 Gew.-%, oder ≥80 Gew.-%, oder ≥90 Gew.-%, oder ≥95 Gew.-%, oder ≥99 Gew.-% betragen kann (selbstredend liegt der Gehalt an (Meth)acrylmonomeren stets bei Werten < 100 Gew.-%). Häufig liegt der (Meth)acrylmonomerengehalt solcher Stoffgemische bei 10 bis 40 Gew.-% oder bei 90 bis 99,5 Gew.-%.

In der Regel werden diese Stoffgemische beim erfindungsgemäßen Verfahren selbst als (Meth)acrylmonomere enthaltender Stoffstrom in den wenigstens einen trennwirksamen Raum geführt.

Häufig umfasst der trennwirksame Raum bei den eingangs beschriebenen thermischen Trennverfahren eine Trennkolonne. Die Anreicherung der (Meth)acrylmonomere kann dabei sowohl am Kopf als auch im Sumpf der Trennkolonne erfolgen. Selbstredend können aber auch im oberen, unteren oder mittleren Teil der Trennkolonne (Meth)-acrylmonomere angereichert enthaltende Fraktionen entnommen werden.

Die im trennwirksamen Raum, z.B. in der Trennkolonne, mitverwendeten trennwirksamen Einbauten verfolgen bei thermischen Trennverfahren den Zweck, die Oberfläche für den die Auftrennung bewirkenden Wärme- und Stoffaustausch zu erhöhen.

Als solche Einbauten kommen z.B. Packungen, Füllkörperschüttungen und/oder Stoffaustauschböden in Betracht.

Besonders häufig werden als Trennkolonnen mit trennwirksamen Einbauten für thermische Trennverfahren solche verwendet, die wenigstens als einen Teil der trennwirksamen Einbauten eine Abfolge von Stoffaustauschböden enthalten.

Stoffaustauschböden verfolgen den Zweck, in der Trennkolonne in Form von Flüssigkeitsschichten Orte mit geschlossenen flüssigen Phasen zur Verfügung zu stellen. Die Oberfläche des in der Flüssigkeitsschicht z.B. aufsteigenden und sich dabei in der geschlossenen flüssigen Phase verteilenden Dampf- bzw. Gasstromes ist dann die maßgebende Austauschfläche. Stoffaustauschböden schließen mit der sie umgebenden Wandung vorzugsweise dicht ab. Ein Klassiker unter den Stoffaustauschböden ist der Siebboden. Darunter sollen in dieser Schrift Platten verstanden werden, die als Durchtrittsstellen für die aufsteigende Gas- bzw. Dampfphase (die Begriffe "gasförmig" und "dampfförmig" werden in dieser Schrift synonym verwendet) einfache Löcher und/oder Schlitze aufweisen.

Die Siebböden werden dabei üblicherweise in zwei Gruppen differenziert, nämlich in solche mit Flüssigkeitszwangsführung und solche ohne Flüssigkeitszwangsführung.

Ganz generell wird Flüssigkeitszwangsführung bei Stoffaustauschböden dadurch erzielt, dass die Stoffaustauschböden wenigstens einen Ablaufschacht aufweisen (Ablauf), durch den die Flüssigkeit unabhängig vom Strömungsweg des Dampfes vom höher gelegenen Boden auf den tiefer gelegenen Boden fließt (Zulauf). Die horizontale Flüssigkeitsströmung über den Austauschboden vom Zulauf zum Ablauf wird entsprechend der verfahrenstechnischen Aufgabenstellung gewählt. Das Gas bzw. der Dampf tritt durch die offenen Querschnitte der Bodenplatte.

Wird die Flüssigkeit im Umkehrstrom über den Boden geführt (Zulauf und Ablauf des Stoffaustauschbodens sind auf der gleichen Seite des Bodens angeordnet), spricht man von Umkehrstromböden. Bei Radialstromböden strömt die Flüssigkeit auf dem Boden radial von der Mitte (Zulauf) zum Ablauf am Rand des Bodens.

Bei den Querstromböden wird die Flüssigkeit, über den gesamten Fließbereich betrachtet, quer über den Boden vom Zulauf zum Ablauf geführt. In der Regel sind Querstromböden einflutig gestaltet. D.h., Zulauf und Ablauf sind auf gegenüberliegenden Seiten des Bodens angeordnet. Sie können aber auch zweiflutig (oder auch mehr als zweiflutig) gestaltet sein. In diesem Fall kann der Zulauf z.B. in der Mitte und je ein Ablauf auf gegenüberliegenden Seiten des Stoffaustauschbodens angeordnet sein.

D.h., bei Siebböden wird die Flüssigkeitszwangsführung dadurch erzielt, dass die Siebböden neben den Durchtrittsstellen für die aufsteigende Gas- bzw. Dampfphase wenigstens einen Ablaufschacht aufweisen (Ablauf), durch den die Flüssigkeit unabhängig vom Strömungsweg des Dampfes vom höher gelegenen Boden auf den nächsten tiefer gelegenen Boden fließt (Zulauf). Die Flüssigkeit fließt z.B. im Querstrom über den Boden vom wenigstens einen Zulauf zum wenigstens einen Ablauf, wobei das Zulauf- und Ablaufrohr den Flüssigkeitsverschluss und die gewünschte Flüssigkeitshöhe auf dem Boden garantieren. Häufig (insbesondere bei geringen Kolonnendurchmessern) sind die Siebböden mit Flüssigkeitszwangsführung einflutig gestaltet. D.h., Zulauf und Ablauf sind auf gegenüberliegenden Seiten des Bodens angeordnet. Sie können aber auch zweiflutig (oder auch mehr als zweiflutig) gestaltet sein. In diesem Fall kann der Zulauf z.B. in der Mitte und je ein Ablauf auf gegenüberliegenden Seiten des Stoffaustauschbodens angeordnet sein. Nachfolgend sollen solche Siebböden als Zwangssiebböden bezeichnet werden. Bei ihnen wird ein die Trennwirkung minderndes Durchregnen der Flüssigkeit nicht wie beim hydraulisch abgedichteten Querstromboden durch Kamine verhindert, in die die Durchtrittsöffnungen fortführen, sondern es bedarf dazu einer minimalen Dampfbelastung. Der Dampf tritt aufsteigend durch die Durchtrittsöffnungen und durchperlt die vom Ablaufrohr gehaltene Flüssigkeitsschicht.

Von den Zwangssiebböden unterscheiden sich die Dual-Flow- oder auch Regensiebböden dadurch, dass sie kein Ablaufsegment enthalten. Durch die Abwesenheit von Ablaufsegmenten (Ablaufschächten) treten bei den Regensiebböden das aufsteigende Gas und die in der Trennkolonne absteigende Flüssigkeit durch die gleichen Durchtrittsstellen des Bodens. Auch beim Regensiebboden bedarf es wie beim Zwangssiebboden einer minimalen Dampfbelastung, um eine angemessene Trennwirkung zu erzielen. Wird sie signifikant unterschritten, bewegen sich aufsteigendes Gas und absteigender Rücklauf im wesentlichen ohne Austausch aneinander vorbei und der Boden läuft Gefahr, trocken zu laufen.

D.h., auch beim Regensiebboden muss eine untere Grenzgeschwindigkeit vorhanden sein, damit auf dem Boden eine gewisse Flüssigkeitsschicht gehalten wird, um ein Arbeiten des Bodens zu ermöglichen. Im normalen Arbeitsbereich regnet die Flüssigkeit bei Regensiebböden durch die Durchtrittsöffnungen von Boden zu Boden und zwischen den Böden wird die geschlossene Gasphase von einer zerteilten Flüssigkeitsphase durchsetzt.

Hydraulisch abgedichtete Querstromböden sind gegenüber Siebböden dadurch charakterisiert, dass sie beim Abschalten der Kolonne nicht leer laufen können, sieht man von der winzigen Leerlaufbohrung (ihr Querschnitt ist normalerweise mehr als 200 mal kleiner als der Gesamtquerschnitt der Durchtrittsstellen) ab, die jeder Querstromboden aus Zweckmäßigkeitsgründen aufweist.

D.h., auch bei geringen Kolonnenbelastungen weisen hydraulisch abgedichtete Querstromböden gestaute Flüssigkeit (Rücklauf- und/oder Zulaufflüssigkeit) auf und laufen keine Gefahr, trocken zu laufen. Dies ist dadurch bedingt, dass es sich bei den Durchtrittstellen von hydraulisch abgedichteten Querstromböden nicht wie bei Siebböden um kaminlose Bohrungen handelt. Vielmehr mündet jede Durchtrittstelle in einen Kamin, der ein Trockenlaufen unterbindet. Über dem Kamin sind Dampfumlenkhauben (Glocken) angebracht, die in die gestaute Bodenflüssigkeit eintauchen. Häufig sind die Dampfumlenkhauben an ihren Rändern geschlitzt oder gezackt (d.h., sie weisen Treibschlitze auf). Der durch die Durchtrittsstelle aufsteigende Dampfstrom erfährt durch die Dampfumlenkhauben eine Ablenkung und strömt parallel zum Boden, d.h., quer zur Kolonne, in die gestaute Flüssigkeit.

Die aus benachbarten, in der Regel über dem Boden äquidistant verteilt angeordneten, Hauben austretenden Dampfblasen bilden in der gestauten Flüssigkeit eine Sprudelschicht aus.

Ablaufrohre bzw. -segmente, die, in der Regel abwechselnd links oder rechts, Böden verlassen, regeln - von Wehren unterstützt - den Flüssigkeitsstand der Stoffaustauschböden und führen die Flüssigkeit dem darunterliegenden Boden zu. Für die hydraulisch abdichtende Wirkung ist wesentlich, dass die Ablaufrohre bzw. -Segmente des oberen Bodens in die gestaute Flüssigkeit des darunter liegenden Bodens tauchen. Vorzugsweise sind keine Zulaufwehre vorhanden. In der Höhe einstellbare Glocken gestatten ein Anpassen an die Strömungsverhältnisse und den Ausgleich der Eintauchtiefen bei Herstellungsungleichmäßigkeiten, so dass alle Glocken des Bodens gleichmäßig gasen.

Je nach Gestalt und Anordnung der Glocken unterscheidet man z.B. die einflutig gestalteten hydraulisch abgedichteten Querstromböden in Rundglockenböden (Durchtrittstelle, Kamin und Glocke sind rund), Tunnel-Böden (Durchtrittstelle, Kamin und Glocke sind rechteckig, die Glocken sind hintereinander angeordnet, wobei die längere Rechteckkante parallel zur Querstromrichtung der Flüssigkeit ausgerichtet ist) und Thormann-Böden (Durchtrittstelle, Kamin und Glocke sind rechteckig, die Glocken sind hintereinander angeordnet, wobei die längere Rechteckkante senkrecht zur Querstromrichtung der Flüssigkeit ausgerichtet ist).

Unter Ventilböden sollen in dieser Schrift Querstromböden verstanden werden, die Bodenbohrungen mit hubbegrenzten Teller-, Ballast - oder Hebeventilen (Schwimmklappen) aufweisen, die die Größe der Dampfdurchtrittsöffnung der jeweiligen Kolonnenbelastung anpassen. Der aufsteigende Gasstrom wird abgelenkt, strömt parallel zum Boden in die gestaute Rücklaufflüssigkeit und bildet eine Sprudelschicht aus. Bewehrte Ablaufrohre führen den Rücklauf von Boden zu Boden. Häufig sind sie zweiflutig gestaltet. Sie können aber auch drei - und mehrflutig (z.B. bis zu achtflutig) gestaltet sein.

Stoffaustauschböden, auf denen Gleichgewicht herrscht zwischen ablaufender Flüssigkeit und aufsteigendem Dampf werden als theoretische Böden bezeichnet.

Dieser Begriff lässt sich sowohl auf alle anderen für Gegenstromdestillationen (Rektifikationen) geeigneten trennwirksamen Einbauten übertragen (wie Packungen und Füllkörperschüttungen) als auch auf andere thermische Trennvorgänge wie die Sorption und Extraktion. Auch bei letzterer bewirken die genannten trennwirksamen Einbauten eine Vergrößerung der Austauschfläche zwischen dann zwei flüssigen Phasen.

Es ist deshalb zweckmäßig, allgemein von theoretischen Trennstufen zu sprechen. Als theoretische Trennstufe wird die Raumeinheit definiert, die eine Anreicherung entsprechend dem thermodynamischen Gleichgewicht bewirkt.

Ziel eines thermischen Trennverfahrens zur Abtrennung wenigstens eines (Meth)acrylmonomere angereichert enthaltenden Stoffstroms aus einem (Meth)acrylmonomere enthaltenden Gemisch ist einerseits ein möglichst hoher Anreicherungsgrad des (Meth)acrylmonomeren im abgetrennten, das (Meth)acrytmonomere angereichert enthaltenden, Stoffstrom und andererseits eine möglichst hohe Raum-Zeit-Ausbeute an diesem abgetrennten Stoffstrom.

Nachteilig an den eingangs beschriebenen thermischen Trennverfahren ist, dass normalerweise diejenigen Maßnahmen, die, unter ansonsten gleichbleibenden Randbedingungen, den Anreicherungsgrad erhöhen, gleichzeitig die Raum-Zeit-Ausbeute mindern (z.B. kann durch eine Erhöhung der Anzahl der theoretischen Trennstufen üblicherweise der *Anreicherungsgrad verbessert werden; bei ansonsten gleichbleiben*den Randbedingungen mindert eine Zunahme der Anzahl der theoretischen Trennstufen jedoch normalerweise die Raum-Zeit-Ausbeute).

Ziel der vorliegenden Erfindung war es, den vorgenannten Zusammenhang zu durchbrechen und ein wie eingangs beschriebenes thermisches Trennverfahren zur Abtrennung wenigstens eines (Meth)acrylmonomere angereichert enthaltenden Stoffstroms aus einem (Methyl)acrylmonomere enthaltenden Gemisch zur Verfügung zu stellen, bei dem Anreicherungsgrad und Raum-Zeit-Ausbeute gleichzeitig erhöht sind.

Demgemäss wurde ein thermisches Trennverfahren zur Abtrennung wenigstens eines (Meth)acrylmonomere angereichert enthaltenden Stoffstroms aus einem (Meth)acrylmonomere enthaltenden Gemisch, umfassend den kontinuierlichen stationären Betrieb wenigstens einer thermischen Trennvorrichtung, die wenigstens einen trennwirksamen Raum mit oder ohne Trennwirksame Einbauten umfasst, in den wenigstens ein (Meth)acrylmonomere enthaltender Stoffstrom hinein und aus dem wenigstens ein (Meth)acrylmonomere enthaltender Stoffstrom herausgeführt wird, wobei sich die durch den trennwirksamen Raum geführten individuellen chemischen Verbindungen, die Michael-Addition der (Meth)acrylmonomeren ausgenommen, beim Durchgang durch den trennwirksamen Raum, jeweils bezogen auf die insgesamt in den trennwirksamen Raum geführte Gesamtmenge der jeweiligen individuellen chemischen Verbindung, zu weniger als 20 mol-% chemisch verändern, mit der Maßgabe dass

- der Stoffstrom, der insgesamt in den trennwirksamen Raum hineingeführt und gedanklich dadurch erzeugt wird, dass man die in den trennwirksamen Raum hineingeführten Einzelstoffströme addiert, X Gew.-% an von (Meth)acrylmonomeren verschiedenen Bestandteilen enthält,

- der Stoffstrom, der mit dem höchsten Gewichtsanteil an (Meth)acrylmonomeren aus dem trennwirksamen Raum herausgeführt wird, Y Gew.-% an von (Meth)acrylmonomeren verschiedenen Bestandteilen enthält,

- das Verhältnis $X : Y \geq 5$ beträgt,

- der trennwirksame Raum, ausgenommen an den Stoffstromzufuhr- und an den Stoffstromausfuhrstellen, von einer festen Phase begrenzt wird und wenigstens einen Umlaufwärmetauscher umfasst,

und

- das im trennwirksamen Raum mit Flüssigphase gefüllte Gesamtvolumen $\geq 1 m^3$ beträgt, wobei die Temperatur der Flüssigphase wenigstens teilweise $\geq 80°C$ beträgt,

gefunden, das dadurch gekennzeichnet ist,
dass bei einer Unterteilung des trennwirksamen Raums in n individuelle Volumenelemente, wobei sich die höchste und die niedrigste Temperatur der in einem einzelnen Volumenelement befindlichen Flüssigphase um nicht mehr als 2°C unterscheiden und das Volumenelement ein im trennwirksamen Raum zusammenhängendes ist,
die Gesamtverweilzeit $t_{ges}$ mit

$$t_{ges} = \sum_{i=1}^{n} \frac{m_{si}}{\dot{m}_i} \cdot 2^A$$

$\leq 20$ h beträgt,
wobei
$A = (T_i - T_o)/10°C$,
$T_o = 100°C$,
$T_i = $ der arithmetische Mittelwert aus der in der Flüssigphase des Volumenelements i bestehenden höchsten und niedrigsten Temperatur in °C,
$m_{si} = $ die im Volumen der im Volumenelement i enthaltenen Flüssigphase insgesamt enthaltene Masse an (Meth)acrylmonomeren,
$\dot{m}_i = $ der aus dem Volumenelement i insgesamt herausgeführte Flüssigphasenmassenstrom, und

$$\sum_{i=1}^{n} = $$ die Summe über alle Volumenelemente i ist, mit der Maßgabe, dass Volumenelemente i mit einer in ihnen

enthaltenen Flüssigphasenmasse $m_i$ und $m_i/\dot{m} \geq 100$ h als Totraum - Volumenelemente ebenso wenig in die Summe über alle Volumenelemente i mit einbezogen werden wie Volumenelement i, die keine Flüssigphase aufweisen und die Gesamtmenge der in den Totraum-Volumenelementen enthaltenen Flüssigphase nicht mehr als 5 Gew.-% der im trennwirksamen Raum insgesamt enthaltenen Flüssigphase beträgt.

Hintergrund der erfindungsgemäßen Verfahrensweise ist der Sachverhalt, dass Methacrylmonomere durch einfache oder mehrfache Michael-Addition Oligomere bzw. Polymere bilden (Michael-Addukte).

Im Fall der Acrylsäure genügen diese beispielsweise der allgemeinen Formel I

$$(y+1) \ CH_2=CH-CO_2H \rightarrow CH_2=CH-CO_2-(CH_2-CH_2-CO_2)_y-H \qquad (I),$$

mit y = insbesondere 1 bis 6, teilweise auch > 6.

Unter anderem ist das Erscheinungsbild der Michael-Addukt Bildung aus der EP-A 733 617, der EP-A 765 861, der DE-A 19 536 191, der DE-A 19 851 984, der DE-A 19 927 722, der EP-A 780 360, der EP-A 780 359, der WO 98/08798, der WO 97/48669 und der DE-A 19 924 533 bekannt.

Nachteil der Michael-Addukt-Bildung von (Meth)acrylmonomeren ist, dass sie auch im Rahmen thermischer Trennverfahren zur Abtrennung eines (Meth)acrylmonomere angereichert enthaltenden Stoffstroms aus einem (Meth) acrylmonomere enthaltenden Gemisch erfolgt und dabei sowohl Anreicherungsgrad als auch Raum-Zeit-Ausbeute mindert. Als mögliche Problemlösung wird im Stand der Technik ledigleich die Isolierung und nachträgliche Rückspaltung der Michael-Addukte gesehen (vgl. z.B. EP-A 780 359, EP-A 780 360, WO 98/08798 und DE-A 19 924 533).

Eingehende Untersuchungen führten als weiterem Problemlösungsbeitrag zum erfindungsgemäßen Verfahren.

Bei diesem Problemlösungsbeitrag findet Berücksichtigung, dass eine Michael-Addukt-Bildung von (Meth) acrylmonomeren im wesentlichen nur in der Flüssigphase erfolgt. Ferner wird berücksichtigt, dass die Michael-Addukt-Bildung in der Flüssigphase sowohl mit zunehmender Temperatur als auch mit zunehmendem Massenanteil der (Meth) acrylmonomeren an der Flüssigphase zunehmend rascher erfolgt, wobei eine Temperaturerhöhung um 10°C etwa eine Verdoppelung der Reaktionsgeschwindigkeit bedingt. Deutlich unterhalb von 100°C ist die Michael-Addition von Acrylmonomeren weitgehend vernachlässigbar.

D.h., wenn man bei kontinuierlich betriebenen thermischen Trennverfahren zur Abtrennung wenigstens eines (Meth)Acrylmonomere angereichert enthaltenden Stoffstroms aus einem (Meth)acrylmonomere enthaltenden Gemisch im stationären Betrieb darauf achtet, dass die Verweilzeit der (Meth)acrylmonomeren in der Flüssigphase dort besonders gering ist, wo Temperatur und Massenanteil an (Meth)acrylmonomeren in der Flüssigphase hoch sind, so lässt sich die nachteilige Michael-Addukt-Bildung von (Meth)acrylmonomeren im Rahmen solcher thermischer Trennverfahren in gewissem Umfang vermeiden.

Ein geeignetes Maß ist diesbezüglich eine aus Einzelverweilzeiten in zweckmäßiger Weise errechnete mittlere Gesamtverweilzeit der (Meth)acrylmonomeren, die diese beim thermischen Trennverfahren im trennwirksamen Raum in flüssiger Phase aufweisen.

Dazu wird der trennwirksame Raum in n individuelle Volumenelemente aufgeteilt. Die Größe und Anzahl n dieser Volumenelemente ist im wesentlichen unbeachtlich. D.h., die einzelnen Volumenelemente können sowohl gleich groß sein, als auch unterschiedliche Volumina aufweisen. Wesentlich ist nur, dass sich die höchste und die niedrigste Temperatur der in einem einzelnen Volumenelement befindlichen Flüssigphase um nicht mehr als 2°C unterscheiden und das Volumenelement ein im trennwirksamen Raum zusammenhängendes ist (mit dem Temperaturerfordernis wird, wie noch zu sehen sein wird, die Temperaturabhängigkeit der Michael-Addition berücksichtigt).

Die Einzelverweilzeit $t_i$ der (Meth)acrylmonomeren im Volumenelement i ist dann gegeben durch $t_i = m_i/\dot{m}_i$. $m_i$ ist dabei die im Volumenelement i insgesamt enthaltene Flüssigphasenmasse und $\dot{m}_i$ ist der aus dem Volumenelement i insgesamt herausgeführte Flüssigphasenmassenstrom (dieser ist im stationären Zustand gleich dem Volumenelement i insgesamt zugeführten Flüssigphasenmassenstrom).

Diese Einzelverweilzeit wird mit dem Quotienten $m_{si}/m_i$ multipliziert, um den Masseanteil der (Meth)acrylmonomeren im Volumenelement i zu berücksichtigen.

Der Faktor $2^A$ mit $A=(T_i-T_o)/10°C$ wichtet die Einzelverweilzeit zusätzlich und trägt der Temperatur in der Flüssigphase des Volumenelementes i Rechnung. Schließlich ist noch über alle Volumenelemente i, die Flüssigphase enthalten, zu summieren.

Totraum-Volumenelemente i sind in die Summe insofern nicht einzubeziehen, als sie am Trennprozeß im wesentlichen nicht beteiligt sind. Dies gilt insbesondere dann, wenn für sie $m_i/\dot{m}_i \geq 200$ h, oder $\geq 300$ h, oder $\geq 400$ h, oder $\geq 500$ h, oder $\geq 750$ h, oder $\geq 1000$ h gilt.

Totraum-Volumenelemente nehmen an den die Trennwirkung bewirkenden Austauschprozessen beim erfindungsgemäßen Trennverfahren im wesentlichen nicht teil. Es handelt sich bei diesen Volumenelementen um im wesentlichen isolierte Volumenelemente, die sich einmal mit Flüssigphase füllen und über die Zeit im wesentlichen ein und dieselbe Flüssigphase enthalten.

Grundsätzlich sind beim erfindungsgemäßen Verfahren Totraum-Volumenelemente weitestgehend zu vermeiden. Dies vor allem auch deshalb, weil sie eine erhöhte Wahrscheinlichkeit des Beginns einer radikalischen Polymerisation der in ihnen enthaltenen (Meth)acrylmonomere aufweisen.

D.h., das erfindungsgemäße Verfahren ist insbesondere dann vorteilhaft, wenn die Gesamtmenge der in den Totraum-Volumenelementen enthaltenen Flüssigphase nicht mehr als 4 Gew.-%, bevorzugt nicht mehr als 3 Gew.-%, besonders bevorzugt nicht mehr als 2 Gew.-% und ganz besonders bevorzugt nicht mehr als 1 Gew.-% beträgt oder sogar verschwindend ist.

Ferner ist für das erfindungsgemäße Verfahren wesentlich, dass X : Y ≥5 beträgt. D.h., die Anzahl der theoretischen Trennstufen wird durch das erfindungsgemäße Verfahren nicht beeinträchtigt. Vielmehr wird man beim erfindungsgemäßen Verfahren die Einzelverweilzeit $t_i$ vor allem dort zurücknehmen, wo einerseits $m_{si}/m_i$ und A groß sind und eine Rücknahme von $t_i$ die Anzahl der theoretischen Trennstufen im wesentlichen nicht beeinträchtigt.

Das erfindungsgemäße Verfahren ist somit auch dann anwendbar, wenn X : Y ≥8, oder ≥10, oder ≥15, oder ≥20, oder ≥30, oder ≥40, oder ≥50 beträgt.

In der Regel wird X : Y beim erfindungsgemäßen Verfahren 200 nicht überschreiten. Meist wird X : Y beim erfindungsgemäßen Verfahren ≤175, vielfach ≤150 oder ≤100 betragen.

Die erfindungsgemäße Verfahrensweise ist vor allem dann vorteilhaft, wenn das im trennwirksamen Raum mit Flüssigphase gefüllte Gesamtvolumen V großvolumig ist. D.h., V kann beim erfindungsgemäßen Verfahren ≥2m³, oder ≥4m³, ≥5m³, oder ≥7m³, oder ≥9m³, ≥10m³, oder ≥15m³ betragen. In der Regel wird V nicht mehr als 500m³ betragen. D.h., in der Regel wird V beim erfindungsgemäßen Verfahren ≤450m³, häufig ≤400m³, oft ≤350m³ und meist ≤300m³ betragen.

Infolge des hohen Siedepunktes von (Meth)acrylmonomeren wird die Temperatur der im trennwirksamen Raum befindlichen Flüssigphase beim erfindungsgemäßen Verfahren häufig wenigstens teilweise ≥90˚C, oder ≥100˚C, oder ≥110˚C, oder ≥120˚C, oder ≥130˚C, oder ≥140˚C, oder ≥150˚C, oder ≥160˚C, oder ≥170˚C, oder ≥180˚C, betragen. In der Regel wird die höchste Temperatur der in einem Volumenelement i befindlichen Flüssigphase beim erfindungsgemäßen Verfahren 250˚C jedoch nicht überschreiten. Im Normalfall wird diese Höchsttemperatur ≥230˚C, häufig ≤210˚C und vielfach ≤200˚C betragen. Eine nennenswerte Rückspaltwirkung vermögen diese Temperaturen in der Regel nicht zu erzielen, da eine solche voraussetzt, dass die Rückspaltprodukte kontinuierlich aus dem Spaltgleichgewicht abgezogen werden.

Mit Vorteil beträgt die Gesamtverweilzeit $t_{ges}$ beim erfindungsgemäßen Verfahren ≤15 h, oder ≤10 h. Mit besonderem Vorteil beträgt $t_{ges}$ erfindungsgemäß ≤8 h, oder ≤6 h bzw. ≤4 h, oder ≤2 h. In der Regel wird $t_{ges}$ beim erfindungsgemäßen Verfahren jedoch ≥0,5 h oder ≥1 h betragen.

Das erfindungsgemäße Verfahren ist u.a. auf das in den Schriften EP-A 648 732 und EP-A 270 999 beschriebene rektifikative Verfahren zur Reinigung von Roh- (Meth)-acrylsäure, auf das in den Schriften DE-A 19 924 533, DE-A 10 247 240 sowie DE-A 10 243 625 beschriebene Verfahren der fraktionierenden Kondensation zur Grundabtrennung von Roh-Acrylsäure aus dem Produktgasgemisch einer Propen- und/oder Propanpartialoxidation zu Acrylsäure, auf das in der EP-A 717 029 beschriebene Verfahren der rektifikativen Abtrennung von Roh- (Meth)acrylsäure aus einem (Meth)acrylsäure und eine höher als (Meth)acrylsäure siedende inerte hydrophobe organische Flüssigkeit als Hauptbestandteil enthaltenden Gemisch, auf die in der DE-A 4 308 087 beschriebene absorptive Abtrennung von Acrylsäure aus den Reaktionsgasen der katalytischen Partialoxidation von Propen und/oder Acrolein sowie auf die anderen in diesen Schriften sowie in der EP-A 990 636, in der EP-A 861 820, in der EP-A 778 255, in der EP-A 551 111, in der EP-A 695 736, in der EP-A 1026145 und in der DE-A 10 251 328 beschriebenen und/oder durch eine Bezugsliteratur zitierten rektifikativen, sorptiven und/oder extraktiven thermischen Trennverfahren zur Abtrennung wenigstens eines (Meth)acrylmonomere angereichert enthaltenden Stoffstroms aus einem (Meth)acrylmonomere enthaltenden Gemisch anwendbar.

Meist umfasst der trennwirksame Raum bei den vorgenannten Verfahren sowie beim erfindungsgemäßen Verfahren ganz generell wenigstens einen Umlaufwärmetauscher und wenigstens eine Kolonne mit oder ohne trennwirksame Einbauten.

Da absorptive Abtrennungen in der Regel exotherm verlaufen, verfolgt der wenigstens eine Umlaufwärmetauscher in diesem Zusammenhang in der Regel den Zweck des Wärmeentzugs. Dieser erfolgt im Normalfall auf indirektem Weg mittels eines Kälteträgers (Kühlmittels), der in den trennwirksamen Raum niemals eintritt. D.h., lediglich eine der beiden Raumseiten des indirekten Wärmetauschers ist Bestandteil des trennwirksamen Raums. Die andere befindet sich außerhalb und führt den Kälteträger. Die Stoffstromförderung durch die zum trennwirksamen Raum gehörige Raumseite des Wärmetauschers erfolgt üblicherweise mittels wenigstens einer Pumpe.

Bei der Mehrzahl der von der absorptiven Abtrennung verschiedenen thermischen Abtrennungen wird über den Umlaufwärmetauscher dem trennwirksamen Raum Wärme zugeführt. Dies erfolgt in den meisten Fällen ebenfalls auf indirektem Weg mittels eines Wärmeträgers, der in den trennwirksamen Raum niemals eintritt. D.h., lediglich eine der beiden Raumseiten des indirekten Wärmetauschers ist Bestandteil des trennwirksamen Raums. Die andere befindet sich außerhalb und führt den Wärmeträger. Häufig wird soviel Wärme zugeführt, dass es in der zum trennwirksamen Raum gehörigen Raumseite des Wärmetauschers zu Siedeerscheinungen kommt.

In diesem Fall bezeichnet man den Umlaufwärmetauscher als einen Umlaufverdampfer. Die Stoffstromförderung durch die zum trennwirksamen Raum gehörige Raumseite des Wärmetauschers kann mittels einer Pumpe (Zwangsumlauf) und/oder durch Naturumlauf erfolgen (letzterer erfolgt infolge des Unterschieds in der Massendichte zwischen erwärmtem und nicht erwärmtem Stoffstrom).

Insbesondere dann, wenn es sich bei dem erfindungsgemäßen thermischen Trennverfahren um eine Rektifikation handelt, wird als Umlaufwärmetauscher ein Umlaufverdampfer eingesetzt.

Prinzipiell können beim erfindungsgemäßen Verfahren als indirekte Umlaufwärmetauscher alle bekannten indirekten Wärmetauschertypen verwendet werden. Vorzugsweise werden sie so gewählt, dass das in ihnen vorgehaltene Flüssigphasenvolumen minimal ist.

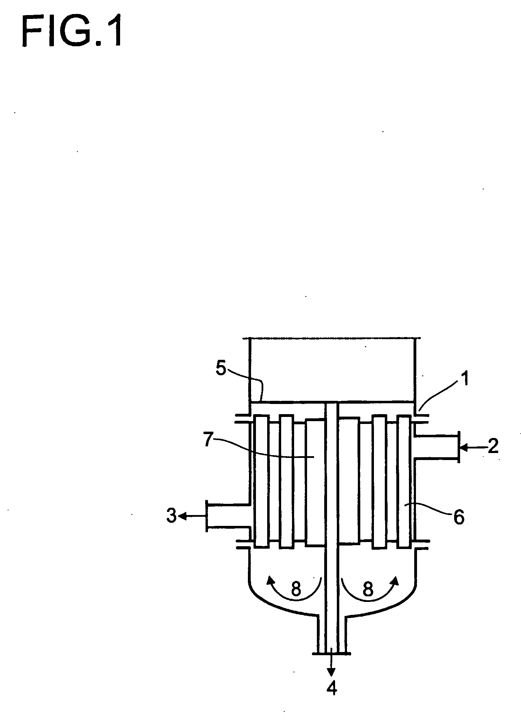

Im Fall eines Umlaufverdampfers kommen z.B. in die Trennkolonne integrierte Robert-Verdampfer (Naturumlaufverdampfer) in Betracht, wie es in Fig. 1 skizziert ist (1=Trennkolonne, 2=Heizdampf, 3=Heizdampfkondensat, 4=Sumpfaustrag, 5=Flüssigkeitsstand; 6=Verdampferrohr, 7=zentrales Fallrohr, 8=Umlaufrichtung).

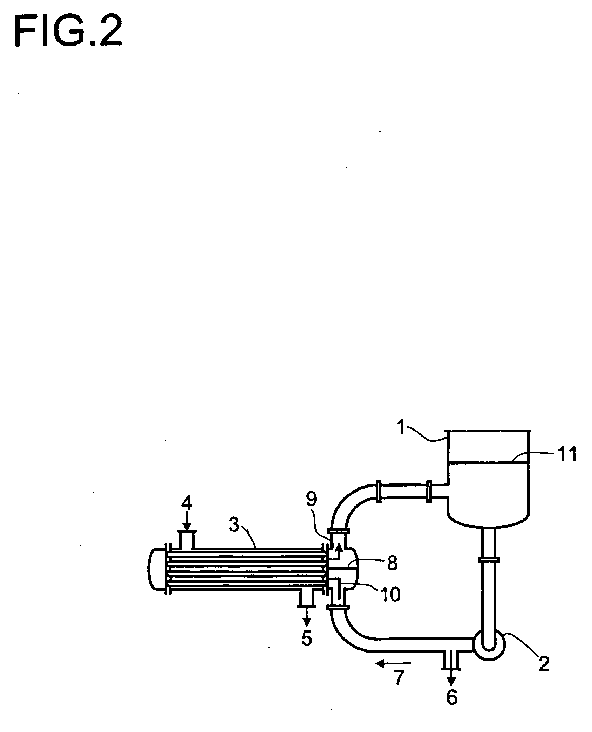

Der Umlaufverdampfer kann aber auch ein aus der Trennkolonne ausgelagerter Zwangsumlaufverdampfer sein, wie ihn Fig. 2 zeigt (1 =Trennkolonne, 2=Umlaufpumpe, 3= Röhrenverdampfer, 4=Heizdampf, 5= Heizdampfkondensat, 6=Sumpfproduktentnahme, 7=Umlaufrichtung, 8=Trennvorrichtung, 9=Ausstrom, 10=Zustrom, 11 =Flüssigkeitsstand).

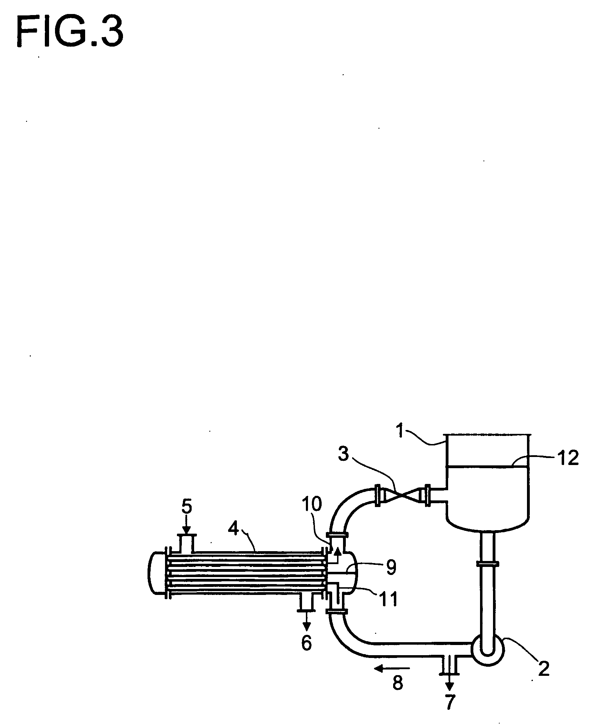

Selbstverständlich kann als Umlaufverdampfer aber auch ein Zwangsumlaufentspannungsverdampfer eingesetzt werden, wie ihn Fig. 3 schematisch zeigt (1=Trennkolonne, 2=Umlaufpumpe, 3=Drosselvorrichtung, 4= Röhrenverdampfer, 5=Heizdampf, 6= Heizdampfkondensat, 7=Sumpfproduktentnahme, 8= =Umlaufrichtung, 9=Trennvorrichtung, 10=Ausstrom, 11=Zustrom, 12=Flüssigkeitsstand). Der Zwangsumlaufentspannungsverdampfer ist im Unterschied zum Zwangsumlaufverdampfer von der Trennkolonne durch eine Drosselvorrichtung getrennt. Dem bei einem Druck $P_x$ befindlichen flüssigen Inhalt der Trennkolonne wird kontinuierlich ein Teil entnommen und mittels einer Umlaufpumpe in die Zuströme eines z.B. Röhrenverdampfers (Rohrbündelwärmeaustauscher) gepumpt. Um die innenliegenden Rohre des Röhrenverdampfers strömt ein Wärmeträger, z.B. Heizdampf (in der Regel unter Druck stehender Wasserdampf), dessen Temperatur oberhalb der Temperatur des Flüssiginhalts der Trennkolonne liegt. Auf dem Weg durch die Zu- und Ausströmrohre des Röhrenverdampfers wird die entnommene Trennkolonnenflüssigkeit durch indirekten Wärmeaustausch auf eine Temperatur $T_{y'}$ erhitzt, die oberhalb der Temperatur des Flüssiginhalts der Trennkolonne liegt.

Eine Drosselvorrichtung trennt Röhrenverdampfer und Trennkolonne druckseitig und ermöglicht durch geeignete Wahl der Umlaufpumpenleistung die Einstellung eines oberhalb von $P_x$ gelegenen Drosselvordrucks $P_y$, der oberhalb des zur Temperatur $T_{y'}$ gehörigen Siededrucks $P_{y'}$ der entnommenen Trennkolonnenflüssigkeit liegt. Durch vorstehende Maßnahmen wird ein Sieden des umgepumpten Trennkolonnenflüssigkeitsanteils in den Röhren des Röhrenverdampfers unterdrückt. Der umgepumpte Anteil der Trennkolonnenflüssigkeit wird in den Rohren des Röhrenverdampfers bezüglich des über dem flüssigen Inhalt der Trennkolonne herrschenden Druck $P_x$ vielmehr überhitzt und der Siedeprozess so auf die Durchtrittseite der Drosselvorrichtung verlagert (d.h., der Inhalt der Röhren des Röhrenverdampfers liegt einphasig vor, der Röhrenverdampfer fungiert lediglich als Überhitzer. Der Durchtritt der überhitzten Flüssigkeit durch die Drosselvorrichtung in die Trennkolonne kann dabei unmittelbar in den flüssigen Inhalt der Trennkolonne (des Trennkolonnensumpfes) hinein erfolgen. Unter diesen Bedingungen entspricht die Temperatur des flüssigen Inhalts des Trennkolonnensumpfes regelmäßig der zum über der Sumpfflüssigkeit herrschenden Druck $P_x$ gehörigen Siedetemperatur $T_x$.

Prinzipiell kann der Durchtritt der überhitzten Flüssigkeit durch die Drosselvorrichtung in die Trennkolonne aber auch oberhalb des Flüssigkeitsspiegels des Trennkolonnensumpfes erfolgen. Unter diesen Bedingungen liegt die Temperatur des flüssigen Inhalts des Trennkolonnensumpfes regelmäßig unterhalb der zum über der Sumpfflüssigkeit herrschenden Druck $P_x$ gehörigen Siedetemperatur $T_x$. Wesentlich ist, dass die Siedeverdampfungswirkung des außerhalb der Trennkolonne angebrachten Röhrenverdampfers erst in der Trennkolonne, d.h., außerhalb des Umlaufverdampfers, eintritt. Die Drosselung kann z.B. mechanisch (Blenden, Ventile) und/oder hydrostatisch (durch eine entsprechend hohe Sumpfsäule über der Durchtrittstelle der überhitzten Flüssigkeit) erfolgen.

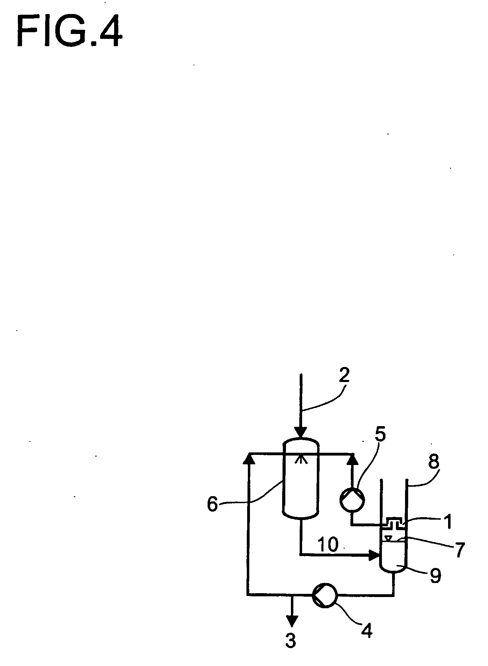

Der Umlaufverdampfer kann aber auch ein Direktumlaufverdampfer sein, wie ihn beispielhaft Figur 4 zeigt, in den der außerhalb des trennwirksamen Raums erzeugte Wärmeträger hineingeführt und mit der zu verdampfenden Flüssigkeit in direkten Kontakt gebracht wird.

Aus dem Sumpf 9 der Trennkolonne 8 wird Sumpfflüssigkeit entnommen und/oder von einem im unteren Teil der Trennkolonne gelegenen Sammelboden 1 wird Schwersiederfraktion entnommen. Entweder eine oder beide Flüssigkeitsentnahmen werden dann in den Direktumlaufverdampfer 6 versprüht, in welchem sie z.B. mit dem eine höhere Temperatur aufweisenden Produktgasgemisch 2 einer Propen- und/oder Propanpartialoxidation als direktem Wärmeträger im Gleichstrom geführt und in direkten Wärmeaustausch mit selbigem gebracht und dabei wenigstens teilweise verdampft werden. Anschließend wird das gesamte Gemisch 10 in die Sumpfflüssigkeit (die Rückführung kann auch nicht getaucht erfolgen) der Trennkolonne rückgeführt und das daraus aufsteigende Gasgemisch in sich selbst aufsteigend fraktionierend kondensiert.

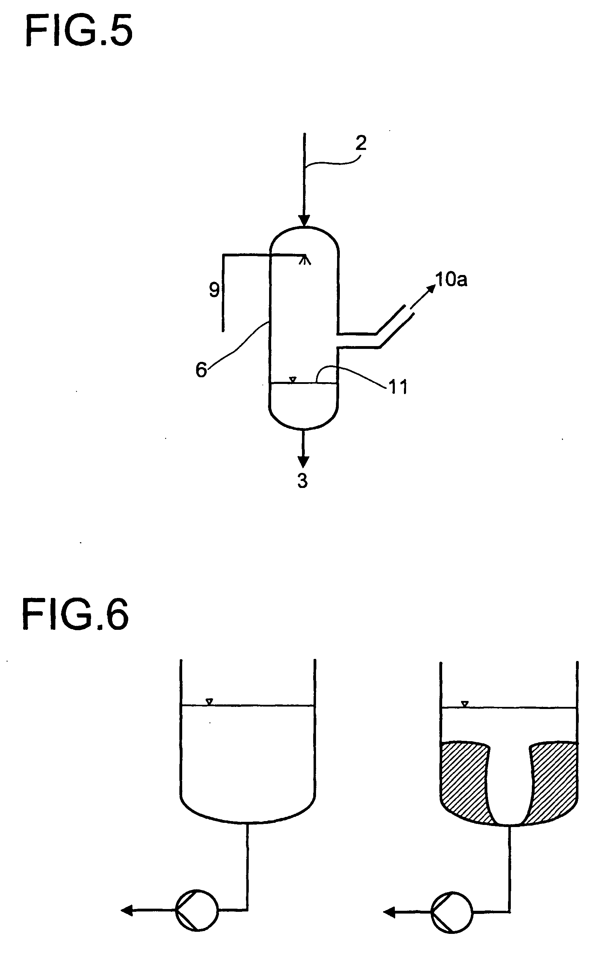

Die Förderung der der Trennkolonne entnommenen Flüssigkeiten erfolgt mittels Pumpen 4, 5. 3 ist die Sumpfproduktentnahme und 7 ist der Stand der Sumpfflüssigkeit. Der Direktumlaufverdampfer ist in der Regel an trennwirksamen Einbauten frei und von zylindrischer Geometrie. Prinzipiell können die Gas- und die Flüssigphase aus dem Direktumlaufverdampfer auch räumlich getrennt in die Trennkolonne rückgeführt werden. Einen solchen Direktumlaufverdampfer zeigt schematisch Figur 5. Die Ziffern besitzen die gleiche Bedeutung wie in Figur 4.

10a ist die in die Trennkolonne rückgeführte Gasphase und 10b ist die in die Trennkolonne rückgeführte Flüssigphase. 11 ist der Flüssigkeitsstand im Direktumlaufverdampfer.

Die Trennkolonne kann entweder an trennwirksamen Einbauten frei sein, oder trennwirksame Einbauten enthalten, wobei zur Durchführung des erfindungsgemäßen Verfahrens prinzipiell alle in dieser Schrift bereits genannten trennwirksamen Einbauten für sich oder gegebenenfalls mit anderen trennwirksamen Einbauten gemeinsam in Betracht kommen.

Als schüttbare Füllkörper kommen dabei z.B. Raschig-Glasringe, Sattelkörper, Maschendrahtringe, V2A-Wendeln, Pall-Ringe und Stedman-Körper sowie Füllkörper der zweiten, dritten und vierten Generation in Betracht. Als Einsatzfüllkörper, deren Durchmesser im wesentlichen dem Kolonneninnendurchmesser gleicht, kommen Packungen der unterschiedlichsten Art in Betracht. Dabei handelt es sich in der Regel um durchlässige, großoberflächige dreidimensionale Metall-, Kunststoff- und/oder Keramiknetzwerke.

Handelt es sich beim erfindungsgemäßen thermischen Trennverfahren um eine fraktionierende Kondensation des Gasgemisches einer ein - oder zweistufigen heterogen katalysierten partiellen Oxidation von Propen und/oder Propan zu Acrylsäure, ist die Trennkolonne vorzugsweise eine solche, die, von unten nach oben, zunächst Dual-Flow-Böden und im Anschluss daran hydraulisch abgedichtete Querstromböden (z.B. Thormann®-Böden oder modifizierte Thormann-Böden) als trennwirksame Einbauten enthält, wie es die DE-A 19924532, die DE-A 10243625 und die DE-A 10 247 240 empfehlen.

Die Anzahl der theoretischen Trennstufen beträgt dabei zweckmäßig 15 bis 30 und vorzugsweise 20. Der Verdampfer ist dabei zweckmäßig ein Direktumlaufverdampfer wie vorstehend beschrieben. Wird in die Kondensationskolonne noch ein Sauerwasserquench integriert, so kommen für diesen Bereich der Kondensationskolonne als trennwirksame Einbauten bevorzugt Ventilböden in Betracht, wie es die DE-A 19924532, die DE-A 10243625 und die DE-A 10 247 240 beschreiben.

Gemäß der allgemeinen Formel für $t_{ges}$ wird $t_{ges}$ beim erfindungsgemäßen Verfahren dann klein, wenn bei großem $\dot{m}_i$ gleichzeitig $m_{si}$ möglichst klein ist. D.h., das erfindungsgemäße Ziel lässt sich u.a. dadurch erreichen, dass man bei im wesentlichen gleichen Stoffströmen und theoretischen Trennstufen die im trennwirksamen Raum im stationären Betriebszustand insgesamt vorgehaltene Flüssigphase minimiert und dies vor allem in denjenigen Volumenelementen, in denen die Flüssigphase eine besonders hohe Temperatur aufweist und deren Minimierung die Anzahl der theoretischen Trennstufe nicht mindert. Unter anderem stehen dazu die nachfolgenden Möglichkeiten zur Verfügung. Eine erste Möglichkeit besteht darin, die Rohrquerschnitte der Rohrleitungen des trennwirksamen Raums durch die (Meth)acrylmonomere enthaltende Flüssigphase geführt wird, auf Kosten eines gewissen Druckverlustes (bei gleichem Volumenstrom) zu verkleinern und die Rohrlänge minimal zu gestalten.

Bei einer wie beschrieben durchzuführenden fraktionierenden Kondensation ist es im Sinne der vorliegenden Erfindung vorteilhaft, wenn außer einem Direktumlaufverdampfer kein weiterer Umlaufwärmetauscher zum trennwirksamen Raum gehört.

D.h., anders als in Fig. 1 und Fig. 2 der DE-A 19924533 bzw. in der Fig. Der DE-A 19924532 und anders als in der DE-A 10 247 240 empfohlen, würde man erfindungsgemäß bevorzugt auf den Umlaufwärmetauscher 8 der vorerwähnten Figuren vollständig verzichten und die Verdampfungswärme ausschließlich aus dem Produktgasgemisch der Partialoxidation (von z.B. Propen und/oder Propan zu Acrylsäure) beziehen. Einen Direktumlaufverdampfer wird man erfindungsgemäß bevorzugt nicht wie in Fig. 5 skizziert sondern wie in Fig. 4 skizziert gestalten, bei dem das Gemisch aus Gas und Flüssigkeit unmittelbar als Zweiphasensystem in die Trennkolonne geführt wird, was im Unterschied zur Verfahrensführung gemäß Figur 5 die Ausbildung von zusätzlichem Flüssigkeitsstand (mit erhöhter Einzelverweilzeit) vermeidet. Gleichzeitig wird man den Querschnitt des Direktumlaufverdampfers bei gleicher Umlaufmenge (der Direktumlaufverdampfer fungiert gleichzeitig als Direktkühler für das Produktgasgemisch der Partialoxidation; aus wärmebilanztechnischen Gründen ist eine bestimmte Mindestumlaufmenge erforderlich) minimal halten, um so die im System des trennwirksamen Raums insgesamt vorgehaltene Flüssigphasemenge minimal zu halten.

Enthält der trennwirksame Raum als trennwirksame Einbauten Stoffaustauschböden, wird man als solche Stoffaustauschböden erfindungsgemäß vorteilhaft Siebböden, besonders bevorzugt Regensiebböden (Dual-Flow-Böden) einsetzen. Da letztere keine Überlaufwehre aufweisen, sind sie mit besonders geringem Flüssigkeitsstand betreibbar und erfindungsgemäß gegenüber hydraulisch abgedichteten Querstromböden bevorzugt. Letztere wird man nur dann einsetzen, wenn die mit Regensiebböden erzielbare Trennwirkung nicht ausreichend ist.

Umfasst der trennwirksame Raum Sammelböden (z.B. als Bestandteil der Trennkolonne), von denen z.B. abgetrennter flüssiger Stoffstrom aus dem trennwirksamen Raum herausgeführt werden kann (z.B. in den Lagertank oder in den Umlaufwärmetauscher), wird man diese erfindungsgemäß mit möglichst geringem Flüssigkeitsstand betreiben. Dies wird beispielsweise dadurch ermöglicht, dass man auf den Sammelböden Verdrängungskörper aufbringt. Diese erlauben ein erhöhtes Flüssigkeitsniveau bei verringertem Flüssigkeitsvolumen. Eine alternative Lösung bietet die DE-A 10159825 in Form von Gefälle aufweisenden Sammelböden an.

Entsprechende Verdrängungskörper erweisen sich beim erfindungsgemäßen Verfahren in der Regel auch in den Sumpfsegmenten (Sumpfräumen) der Trennkolonnen als vorteilhaft. Dies sind in der Regel jene Raumanteile, die sich geometrisch unterhalb der untersten trennwirksamen Einbauten befinden. Vor allem im Fall einer Rektifikation als erfindungsgemäßem Trennverfahren wird aus den Sumpfsegmenten regelmäßig Sumpfflüssigkeit abgezogen, um selbige beispielsweise Zwangsumlaufwärmetauschern zuzuführen. Gleichzeitig befindet sich die Sumpfflüssigkeit im Regelfall im Siedezustand.

Um zu vermeiden, dass die beim Zwangsumlaufwärmetauscher zur Zwangsförderung erforderliche Pumpe z.B. im Fall von Betriebsstörungen zuviel Gas zieht (dies mindert in der Regel die Förderleistung der Pumpe und kann in ungünstigen Fällen deren Zerstörung verursachen, da die Pumpe im Normalfall lediglich für eine Flüssigkeitsförderung ausgelegt ist) wird im Sumpfsegment üblicherweise eine Sicherheitsflüssigkeitshöhe, ein Sicherheitsflüssigkeitsstand, eingestellt.

Diese Sicherheitshöhe ist erfindungsgemäß dadurch vorteilhaft mit verringertem Sumpfvolumen erreichbar, dass man im Sumpfraum Verdrängungskörper anbringt oder den Sumpfraum einzieht, wie es z.B. Figur 6 zeigt. Selbstredend kann diese Minderung des Sumpfvolumens beim erfindungsgemäßen Verfahren ganz generell angewendet werden.

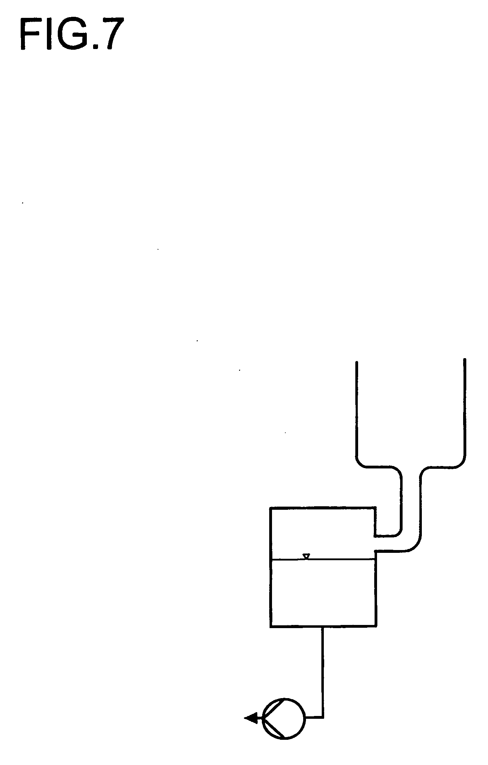

Sie ist erfindungsgemäß in jedem Fall gegenüber solchen Lösungen vorzuziehen, wie sie Figur 7 zeigt, wo zwischen Pumpe und Kolonnensumpf aus Sicherheitsgründen ein Sumpfflüssigkeitspuffergefäß geschaltet ist. Beim erfindungsgemäßen Verfahren mitverwendete Pumpen werden zweckmäßig so gewählt, dass sie eine möglichst geringe Flüssigphasenmenge vorhalten.

Die Bestimmung der Temperaturen $T_i$ ist beim erfindungsgemäßen Verfahren in einfacher Weise durch experimentelle Bestimmung zugänglich (z.B. mittels in geeigneter Weise angebrachten Thermoelementen).

Prinzipiell ist die Wahl der Volumenelemente i beim erfindungsgemäßen Verfahren beliebig und beeinflusst das Ergebnis für $t_{ges}$ im wesentlichen nicht. In vielen Fällen gibt es jedoch eine besonders zweckmäßige Auswahl der Volumenelemente i.

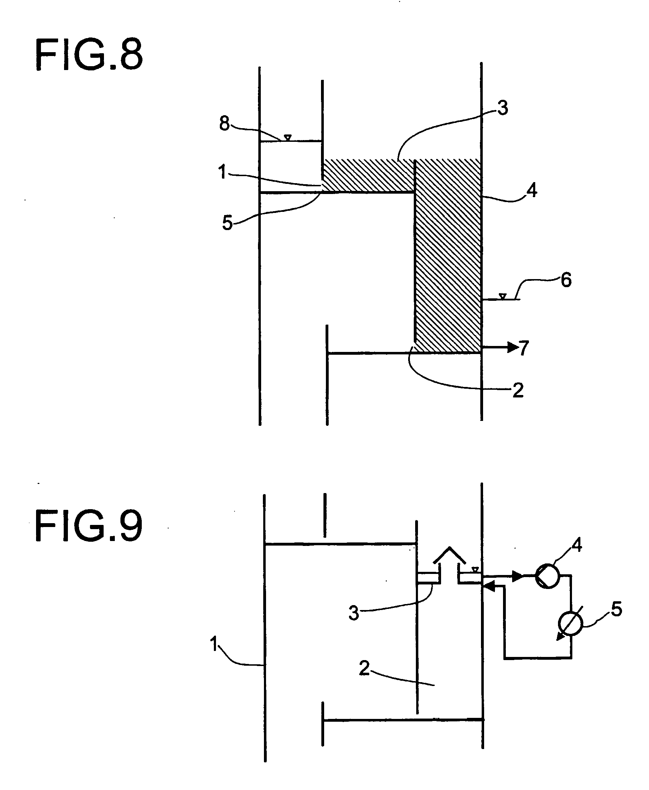

Im Fall von Stoffaustauschböden mit Zwangsführung wird in zweckmäßiger Weise als Volumenelement i dasjenige Volumenelement gewählt, das den Flüssigkeitsstand auf dem Stoffaustauschboden und den Zulauf zum darunter liegenden Stoffaustauschboden erfasst, wie es in Fig. 8 schematisch durch die schraffierte Fläche dargestellt ist. 1 ist die Zuflussstelle zum Volumenelement, 2 ist die Ausflussstelle aus dem Volumenelement, 3 ist das Überlaufwehr, 4 ist die Kolonnenwand und 5 ist der die Durchtrittstellen aufweisende Stoffaustauschboden (6 = Flüssigkeitsstand im Ablauf- bzw. Zulaufschacht).

$m_{si}$ lässt sich z.B. dadurch bestimmen, dass im Betrieb befindlich Zufluss- und Ausflussstelle simultan geschlossen werden und nachfolgend die im Volumenelement i enthaltene Flüssigphasenmasse $m_i$ bestimmt wird. Mittels chemischer Gehaltsanalyse resultiert aus $m_i$ schließlich $m_{si}$. $\dot{m}_i$ lässt sich z.B. dadurch ermitteln, dass man in den Ablaufschacht einen Fangboden einbaut, den demselben zugeführten Flüssigkeitsstrom aus der Trennkolonne herausführt, bestimmt, und unterhalb der Ausfuhrstelle (Ausflussstelle) in den Ablaufschacht rückführt (vgl. Figur 9; 1=Kolonnenwand; 2=Ablaufschacht; 3=Fangboden; 4= Förderpumpe; 5=Strommessung). Alternativ kann man $\dot{m}_i$ bei Kenntnis von $m_i$ auch dadurch bestimmen, dass man an der Zuflussstelle einen Tracer aufgibt und dessen Auftreten an der Ausflussstelle über die Zeit verfolgt. Letzteres kann z.B. dadurch erfolgen, dass man an der Stelle mit der Ziffer 7 in Figur 8 kontinuierlich eine kleine Probe entnimmt. Die Traceraufgabestelle wäre zweckmäßigerweise die Stelle mit der Ziffer 8 in Figur 8.

Im Fall von Regensiebböden kann in entsprechender Weise vorgegangen werden. Hier wird in zweckmäßiger Weise als Volumenelement i dasjenige Volumenelement gewählt, das den Flüssigkeitsstand auf dem Stoffaustauschboden und den Raum unterhalb des Stoffaustauschbodens bis zur beginnenden Oberfläche des Flüssigkeitsstands des nachfolgenden Stoffaustauschbodens erfasst.

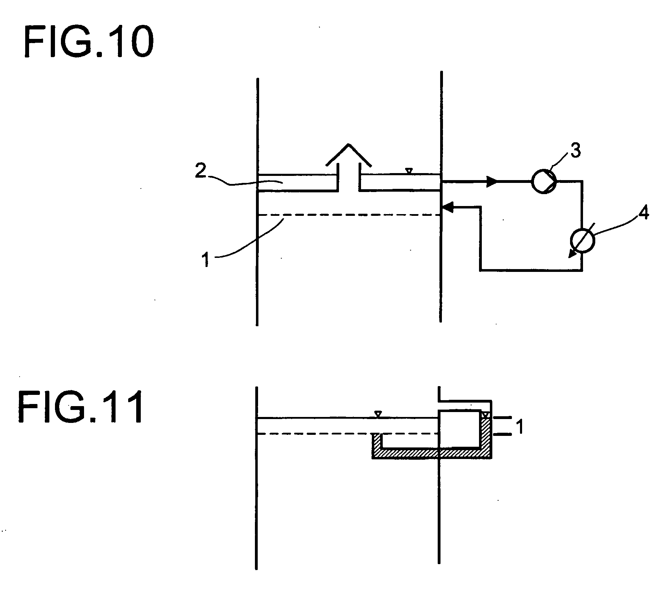

Um in diesem Fall $\dot{m}_i$ zu ermitteln, kann oberhalb des Siebbodens wiederum ein Fangboden angebracht werden. Von diesem wird die zulaufende Flüssigkeitsmenge herausgeführt, der entsprechende Strom bestimmt und anschließend erfolgt unmittelbar oberhalb des Siebbodens die Rückführung (vgl. Figur 10; 1 =Regensiebboden; 2=Fangboden; 3=Förderpumpe; 4=Strommessung).

Die auf dem Regensiebboden befindliche Flüssigkeitsmenge kann beispielsweise mittels einer Differenzdruckmessung gemäß Figur 11 bestimmt werden (U-Rohr-Manometer-Methode). Infolge $\Delta p = sg \cdot h_L$ ($h_L$=1 in Fig. 11), wobei die Massendichte der Flüssigphase, g die Erdbeschleunigung und $h_L$ der Flüssigphasenstand auf dem Regensiebboden ist, lässt sich $h_L$ unmittelbar ablesen und die Flüssigkeitsmenge auf dem Regensiebboden errechnen.

Die Flüssigkeitsmenge zwischen zwei Regensiebböden lässt sich dadurch bestimmen, dass man die beiden Regensiebböden simultan verschließt und die sich auf dem unteren Boden ansammelnde Flüssigkeitsmenge bestimmt. Mittels chemischer Analyse ergibt sich aus $m_i$ schließlich $m_{si}$.

In völliger Entsprechung resultiert $m_{si}$ im Kolonnensumpf aus der durch Ablauf ermittelten Sumpfstandmenge sowie Analyse der Zusammensetzung der Sumpfflüssigkeit. $\dot{m}_i$ lässt sich bei standgeregeltem Sumpf unmittelbar durch Messung ermitteln. Für die Umlaufpumpe gilt das gleiche und der Umlaufwärmetauscher kann ebenso behandelt werden. Kolonnenabschnitte mit schüttbaren Füllkörpern oder mit Einsatzfüllkörpern sind analog behandelbar wie Stoffaustauschbodenvolumenelemente.

Innerhalb einer Trennkolonne lassen sich die $m_{si}$- und $m_i$-Werte aber auch in sehr guter Näherung halbempirisch bestimmen (vgl. z.B. Johann Stichlmair; Grundlagen der Dimensionierung des Gas/Flüssigkeit-Kontaktapparates, Bodenkolonne, Verlag Chemie (1978) und Technische Fortschrittsberichte, Bd. 61, Grundlagen der Dimensionierung von Kolonnenböden, von Dr.-Ing. Klaus Hoppe und Dr.-Ing. Manfred Mittelstrass, Magdeburg, Verlag Theodor Steinkopff, Dresden (1967)).

Dazu wird zunächst z.B. über die verschiedenen Stoffaustauschböden die jeweilige Bodentemperatur ermittelt. Dann wird die Zusammensetzung von Zuläufen und Abläufen aus der Trennkolonne bestimmt. Über das Raoult'sche Gesetz (Dampf-Flüssig-Gleichgewicht) kombiniert mit Massen-Energie-Bilanzen lassen sich dann die Konzentrationsverläufe in der Kolonne errechnen. Aus diesen resultieren dann die benötigten Flüssigkeits- und Gasmengenströme. Die $m_{si}$-Werte lassen sich wie folgt ermitteln. Zunächst wird in separaten Versuchen (von unten wird mit Gas beströmt und von oben wird Flüssigkeit aufgegeben) das hydrodynamische Verhalten der trennwirksamen Einbauten, z.B. der Stoffaustauschböden, ermittelt. Daraus erwachsen die $m_i$-Werte, aus denen mittels der Konzentrationsverläufe schließlich die $m_{si}$-Werte folgen.

Das überraschende Ergebnis der erfindungsgemäßen Verfahrensweise besteht darin, dass sich durch ein Absenken von $t_{ges}$ unter ansonsten im wesentlichen gleichbleibenden Randbedingungen sowohl die Raum-Zeit-Ausbeute als auch der Anreicherungsgrad verbessern lassen.

Selbstverständlich kann die erfindungsgemäße Verfahrensweise mit einer Verfahrensweise kombiniert werden, die eine Rückspaltung der Michel-Addukte integriert enthält. An einigen Stellen dieser Schrift wurden beispielhaft Ausführungen spezifisch auf Acrylsäure als (Meth)acrylmonomeres bezogen. Diese Ausführungen besitzen aber in der Regel auch für die anderen (Meth)acrylmonomeren Gültigkeit.

Wie im nachfolgenden Ausführungsbeispiel gezeigt wird, gestattet es die erfindungsgemäße Verfahrensweise bei einem $t_{ges}$-Wert von ≤10 h sogar, aus dem Produktgasgemisch einer (ein oder zweistufigen) heterogen katalysierten Propen- und/oder Propanpartialoxidation zu Acrylsäure, dessen Acrylsäuregehalt 5 bis 15 Gew.-% beträgt, eine rohe Acrylsäure abzutrennen, deren Acrylsäuregehalt bei Werten von ≥95 Gew.-% liegt.

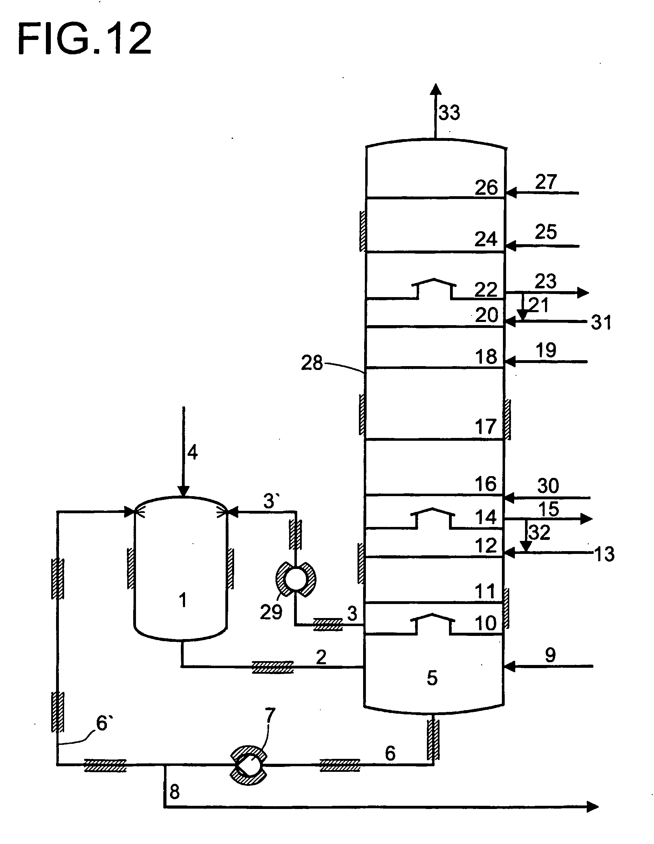

Das dabei anzuwendende thermische Trennverfahren umfasst den kontinuierlichen stationären Betrieb einer thermischen Trennvorrichtung, die als trennwirksamen Raum einen solchen mit trennwirksamen Einbauten umfasst, wie ihn schematisch Figur 12 zeigt.

Er besteht aus einer Trennkolonne 28 (die als trennwirksame Einbauten nur Stoffaustauschböden enthält; bei diesen handelt es sich von unten nach oben zunächst um Dual-Flow-Böden und im Anschluß daran um hydraulisch abgedichtete Querstromböden, die abschließend von Ventilböden abgelöst werden; zusätzlich enthält die Trennkolonne noch Sammelböden), einem an Einbauten freien Direktumlaufverdampfer 1 der mittels einer Pumpe 7 und einer Pumpe 29 fördert sowie Rohrleitungen 6,6', 3,3' und 2. Eine ausführlich Beschreibung einer solchen Trennkolonne findet sich in den Schriften DE-A 19 924 532, DE-A 10 247 240 und DE-A 10 243 625. Die Anzahl der theoretischen Trennstufen beträgt anwendungstechnisch zweckmäßig 15 bis 30 und vorzugsweise 20. Alle Elemente jenseits dieses trennwirksamen Raums können zwar Bestandteil der insgesamt angewandten thermischen Trennvorrichtung sein. Sie gehören jedoch nicht dem erfindungsgemäßen Bilanzraum, dem erfindungsgemäß zu betrachtenden trennwirksamen Raum, an. Eine Erweiterung desselben erhöht $t_{ges}$ und trägt zur zu betrachtenden thermischen Abtrennung nicht bei. Vom erfindungsgemäßen Verfahren wird also bereits dann Gebrauch gemacht, wenn das insgesamt angewandte thermische Trennverfahren ein erfindungsgemäßes Verfahren umfasst. Das aufzutrennende Produktgasgemisch der (ein- oder zweistufigen) heterogen katalysierten Propen- und/oder Propanpartialoxidation zu Acrylsäure ist gleichzeitig direkte Wärmequelle des Direktumlaufverdampfers.

Das nachfolgende Beispiel und Vergleichsbeispiel erläutern die vorliegende Erfindung beispielhaft ohne Beschränkung ihrer Allgemeingültigkeit. Ganz generell enthalten die beim erfindungsgemäßen Verfahren auftretenden (Meth)acrylmonomere umfassenden Flüssigphasen in an sich bekannter Weise Polymerisationsinhibitoren zugesetzt.

Beispiel und Vergleichsbeispiel (die numerischen Adressen beziehen sich auf Figur 12; der in Figur 12 grau schraffiert unterlegte Bereich ist der zu betrachtende trennwirksame Raum)

Vergleichsbeispiel (beschrieben wird der stationäre Zustand; geeignete Materialien sind z.B. Edelstahl der Sorten 1.4539 bzw. 1.4571)

Aus einer heterogen katalysierten Gasphasenpartialoxidation von Propylen der Reinheit "polymer grade" wurde ein eine Temperatur von 270°C aufweisendes Produktgasgemisch der nachfolgenden Gehalte erhalten:

| | |
|---|---|
| 11,80 | Gew.% Acrylsäure, |
| 0,264 | Gew.-% Essigsäure, |
| 5,0984 | Gew.-% Wasser, |
| 0,0275 | Gew.-% Ameisensäure, |
| 0,0989 | Gew.-% Formaldehyd, |

(fortgesetzt)

| | |
|---|---|
| 0,1473 | Gew.-% Acrolein, |
| 0,0028 | Gew.-% Propionsäure, |
| 0,0033 | Gew.-% Furfurale, |
| 0,0014 | Gew.-% Allylacrylat, |
| 0,0005 | Gew.-% Allylformiat, |
| 0,0038 | Gew.-% Benzaldehyd, |
| 0,1350 | Gew.% Maleinsäureanhydrid, |
| 0,0112 | Gew.-% Benzoesäure, |
| 0,0147 | Gew.-% Phthalsäureanhydrid, |
| 4,0324 | Gew.-% Sauerstoff, |
| 1,8067 | Gew.-% Kohlendioxid, |
| 0,5904 | Gew.-% Kohlenmonoxid, |
| 0,5520 | Gew.-% Propan, |
| 0,2696 | Gew.-% Propylen, und |
| 75,1399 | Gew.-% Stickstoff. |

Weitere Bestandteile werden nicht detektiert. Das Produktgasgemisch (170008 kg/h) wird in einem im Gleichstrom betriebenen Direktumlaufwärmetauscher 1 auf eine Temperatur von 120,2˚C abgekühlt. Der im Gleichstrom betriebene Direktumlaufwärmetauscher 1 ist frei von Einbauten. Er weist eine zylindrische Form auf. Sein Durchmesser beträgt 2,2 m und seine Höhe beträgt 15,5 m. Die im Direktumlaufverdampfer 1 zu erhitzende Flüssigphase ist ein Gemisch aus dem Sumpfraum 5 entnommener Sumpfflüssigkeit und aus Schwersiederfraktion, die dem den Sumpfraum 5 der Trennkolonne 28 abschließenden ersten Fangboden 10 entnommen wird.

Die Menge der in den Direktumlaufwärmetauscher 1 geführten Sumpfflüssigkeit beträgt 247305 kg/h und weist folgende Gehalte auf (Massendichte = 989,22 kg/m$^3$):

| | |
|---|---|
| 27,2977 | Gew.-% Acrylsäure, |
| 0,1446 | Gew.-% Essigsäure, |
| 0,6007 | Gew.-% Wasser, |
| 0,0069 | Gew.-% Ameisensäure, |
| 0,0007 | Gew.-% Formaldehyd, |
| 0,0087 | Gew.-% Acrolein, |
| 0,0149 | Gew.-% Propionsäure, |
| 0,2041 | Gew.-% Furfurale, |
| 0,0008 | Gew.-% Allylacrylat, |
| 0,0001 | Gew.-% Allylformiat |
| 0,2490 | Gew.-% Benzaldehyd, |
| 4,4377 | Gew.-% Maleinsäureanhydrid, |
| 0,7354 | Gew.-% Benzoesäure, |
| 0,9605 | Gew.-% Phthalsäureanhydrid, |
| 19,5513 | Gew.-% Diacrylsäure, |
| 40,1375 | Gew.-% Polyacrylsäure (Michael-Addukt), |
| 0,4855 | Gew.-% Phenothiazin, |
| 0,5560 | Gew.-% Monomethylether des Hydrochinons (MEHQ), |
| 4,6079 | Gew.% sonstige hochsiedende Bestandteile, und |
| 0,0002 | Gew.-% Sauerstoff. |

Die Temperatur der Sumpfflüssigkeit beträgt 118,3˚C. Der Dampfdruck liegt bei 1,48 bar. Die Menge der in den Direktumlaufverdampfer 1 geführten Schwersiederfraktion beträgt 63009 kg/h und enthält folgende Gehalte (Massendichte = 969,94 kg/m$^3$):

| | |
|---|---|
| 90,4867 | Gew.-% Acrylsäure, |
| 0,3672 | Gew.-% Essigsäure, |

(fortgesetzt)

| 1,4207 | Gew.-% Wasser, |
| 0,0142 | Gew.-% Ameisensäure, |
| 0,0016 | Gew.-% Formaldehyd, |
| 0,0109 | Gew.-% Acrolein, |
| 0,0535 | Gew.-% Propionsäure, |
| 0,6232 | Gew.-% Furfurale, |
| 0,0025 | Gew.-% Allylacrylat, |
| 0,0002 | Gew.-% Allylformiat, |
| 0,5317 | Gew.-% Benzaldehyd, |
| 4,9046 | Gew.-% Maleinsäureanhydrid, |
| 0,0401 | Gew.-% Benzoesäure, |
| 0,0344 | Gew.-% Phthalsäureanhydrid, |
| 1,4102 | Gew.-% Diacrylsäure, |
| 0,0201 | Gew.-% Phenothiazin, |
| 0,0779 | Gew.-% MEHQ, und |
| 0,0004 | Gew.-% Sauerstoff. |

Die Temperatur der Schwersiederfraktion beträgt 100,4˚C. Der Dampfdruck liegt bei 1,48 bar.

Die Zufuhr der Schwersiederfraktion in den Direktumlaufverdampfer 1 erfolgt über eine Rohrleitung 3 des Durchmessers 150 mm und einer Länge von 10 m zu einer Kreiselpumpe 29 (Flüssigkeitsinhalt: 50 l) und von dort über eine Rohrleitung 3' des Durchmessers 150 mm und einer Länge von 15 m in den Direktumlaufverdampfer 1 (alternativ könnte die Schwersiederfraktion auch durch Überlauf in den Sumpfraum geführt und als Bestandteil der Sumpfflüssigkeit in den Direktumlaufverdampfer geführt werden).

Die Sumpfflüssigkeit, die dem Sumpfraum 5 entnommen wird, wird in einer Menge von 249905 kg/h über eine Rohrleitung 6 mit einem Durchmesser von 300 mm und einer Länge von 10 m einer Kreiselpumpe 7 (Flüssigkeitsinhalt: 100 l) zugeführt und von dort in einer Menge von 247305 kg/h über eine Rohrleitung 6' von 10 m Länge und 300 mm Durchmesser (Rohrleitungsdurchmesser meinen hier stets den Innendurchmesser) in den Direktumlaufverdampfer 1 geführt. 2300 kg/h der entnommenen Sumpfflüssigkeit werden der Rückspaltung zugeführt und 300 kg/h der entnommenen Sumpfflüssigkeit werden dem im folgenden noch zu beschreibenden Quenchkreis I zugeführt, um diesen gegen unerwünschte Polymerisation zu inhibieren (in beiden Fällen erfolgt die Zuführung über die Rohrleitung 8). Das Gemisch aus Sumpfflüssigkeit und Schwersiederfraktion wird im Direktumlaufverdampfer über eine Meisterdüse (Pralldüse gemäß WO 02/50011) versprüht.

Das den Direktumlaufverdampfer 1 mit einer Temperatur von 120,2˚C verlassende Zweiphasengemisch wird über die Rohrleitung 2 (Durchmesser: 1500 mm; Länge : 10 m) in den Sumpfraum 5 rückgeführt.

Der Druck im Sumpfraum 5 und im Direktumlaufverdampfer 1 beträgt 1,48 bar. Die Höhe der Trennkolonne 28 (eine Kolonne zur fraktionierenden Kondensation, die als trennwirksame Einbauten nur Stoffaustauschböden enthält; bei diesen handelt es sich von unten nach oben zunächst um Dual-Flow-Böden und im Anschluß daran um hydraulisch abgedichtete Querstromböden (Thormann-Böden), die abschließend von Ventilböden abgelöst werden) beträgt 54,3 m.

Der Innendurchmesser der Trennkolonne 28 beträgt im Bereich der Thormann-Böden 6,5 m und ansonsten 6,0 m.

Die Rückspaltvorrichtung (die z.B. nicht zum trennwirksamen Raum gehört), der die 2300 kg/h der aus dem Sumpfraum 5 entnommenen Sumpfflüssigkeit zugeführt werden, besteht aus einem Zwangsumlaufentspannungsverdampfer und einer auf diesen nahtlos aufgesetzten Dual-Flow-Boden Rektifikationskolonne. Die Anzahl der Dual-Flow-Böden beträgt 50.

Der Zwangsumlaufentspannungsverdampfer besteht aus einem Spaltkessel, einem Wärmetauscher, einer Pumpe und den zugehörigen Rohrleitungen. Der Sumpfaustrag aus dem Spaltkessel wird über eine Rohrleitung einer Kreiselpumpe zugeführt, die ihn einem Rohrbündelwärmetauscher zuleitet. Anschließend wird ein Teil der erhitzten Flüssigkeit über eine Rohrleitung in den Spaltkessel zurückgefahren. Der andere Teil der erhitzten Flüssigkeit wird Viskositäts- (bevorzugt), Dichte- oder Temperaturgeregelt in einen im folgenden zu beschreibenden Behälter gefahren, in welchem Methanol zugemischt wird.

Ebenso wie die Trennkolonne 28 ist die Rektifikationskolonne gegen die Umgebung isoliert. Der Innendurchmesser der Rektifikationskolonne beträgt über alle Dual-Flow-Böden einheitlich 2,4 m. Ihre Höhe liegt bei 27 m. Die Dual-Flow-Böden sind in der Rektifikationskolonne äquidistant (400 mm) angeordnet. Ihr Öffnungsverhältnis beträgt einheitlich 12 %. Von unten nach oben betrachtet beträgt der Lochdurchmesser der ersten acht Dual-Flow-Böden einheitlich 25 mm (Lochanordnung entsprechend strenger Dreiecksteilung und der Lochdurchmesser aller nachfolgenden Dual-Flow-Böden liegt einheitlich bei 14 mm (Lochanordnung ebenfalls entsprechend strenger Dreiecksteilung). Die Zufuhr der der Rückspaltung zu unterwerfenden Sumpfflüssigkeit erfolgt auf den achten Dual-Flow-Boden (von unten).

Dem Spaltgefäß des Zwangsumlaufentspannungsverdampfers werden 20000 kg/h von am Kopf der Kondensationskolonne abgeführtem, nachfolgend überhitztem und komprimierten Kreisgas (als Unterstützungsgas) zugeführt (Druck = 2,9 bar; Temperatur = 160°C).

Die Gehalte des Kreisgases lauten:

| | |
|---|---|
| 0,2288 | Gew.-% Acrylsäure, |
| 0,0885 | Gew.-% Essigsäure, |
| 2,6689 | Gew.-% Wasser, |
| 0,0052 | Gew.-% Ameisensäure, |
| 0,1724 | Gew.-% Acrolein, |
| 0,0002 | Gew.-% Propionsäure, |
| 0,0003 | Gew.-% Furfurale, |
| 0,0012 | Gew.-% Allylformiat, |
| 4,7392 | Gew.-% Sauerstoff, |
| 2,1235 | Gew.-% Kohlendioxid, |
| 0,6939 | Gew.-% Kohlenmonoxid, |
| 0,6487 | Gew.-% Propan, |
| 0,3169 | Gew.-% Propylen und |
| 88,3123 | Gew.-% Stickstoff. |

Dem Spaltgefäß des Zwangsumlaufentspannungsverdampfers werden stetig 522963 kg/h Flüssigphase mit einer Temperatur von 161°C bei einem Druck von 1,71 bar entnommen. Davon werden 522246 kg/h nach Durchlaufen des Wärmetauschers mit einer Temperatur von 166°C und einem Druck von 3 bar in das Spaltgefäß rückgeführt. Die anderen 717 kg/h davon werden entgast und mit Methanol verdünnt der Rückstandsverbrennung zugeführt.

Die im Spaltgefäß sich bildenden Spaltgase werden durch das zugeführte Unterstützungsgas in die aufsitzende Rektifikationskolonne gefördert und steigen in dieser in absteigender Rücklaufflüssigkeit auf.

Aus dem Kopf der Rektifikationskolonne wird in einer Menge von 33129 kg/h ein Gasgemisch (umfassend Kreisgas und Spaltgas) mit einer Temperatur von 99,8°C und einem Druck von 1,60 bar herausgeführt und einem im Gleichstrom betriebenen Sprühkühler (Quenchkreis I) durch direkte Kühlung auf eine Temperatur von 63,3°C abgekühlt und partiell kondensiert.

Das nach der Direktkühlung verbleibende Gasgemisch wird in einer Menge von 21883 kg/h mit nachfolgenden Gehalten über die Leitung 9 in den Sumpfraum 5 der Kondensationskolonne 28 rückgeführt (nicht getaucht):

| | |
|---|---|
| 8,7215 | Gew.-% Acrylsäure, |
| 0,0976 | Gew.-% Essigsäure, |
| 2,5067 | Gew.-% Wasser, |
| 0,0056 | Gew.-% Ameisensäure, |
| 0,0001 | Gew.-% Formaldehyd, |
| 0,1584 | Gew.-% Acrolein, |
| 0,0019 | Gew.-% Propionsäure, |
| 0,0017 | Gew.-% Furfurale, |
| 0,0001 | Gew.-% Allylacrylat, |
| 0,0011 | Gew.-% Allylformiat, |
| 0,0004 | Gew.-% Benzaldehyd, |
| 0,0039 | Gew.-% Maleinsäureanhydrid, |
| 4,3313 | Gew.-% Sauerstoff, |
| 1,9407 | Gew.-% Kohlendioxid, |
| 0,6342 | Gew.-% Kohlenmonoxid, |
| 0,5929 | Gew.-% Propan, |
| 0,2896 | Gew.-% Propylen und |
| 80,7122 | Gew.-% Stickstoff. |

Als Quenchflüssigkeit I wird ein Gemisch aus den 300 kg/h dem Sumpfraum 5 entnommener Sumpfflüssigkeit und bei der Direktkühlung im Quenchkreis I gebildetem Kondensat verwendet. 104207 kg/h dieses Gemisches werden durch indirekte Kühlung auf 32°C abgekühlt und im Sprühkühler I des Quench I versprüht. 11546 kg/h desselben Gemisches werden mit einer Temperatur von 63,3°C auf den obersten Dual-Flow-Boden der dem Spaltgefäß aufsitzenden Rektifikationskolonne als Rücklaufflüssigkeit rückgeführt.

Die Zusammensetzung der Quenchflüssigkeit I lautet:

|  |  |
|---|---|
| 93,7485 | Gew.-% Acrylsäure, |
| 0,4937 | Gew.-% Essigsäure, |
| 3,7513 | Gew.-% Wasser, |
| 0,0143 | Gew.-% Ameisensäure, |
| 0,0328 | Gew.-% Acrolein, |
| 0,0207 | Gew.-% Propionsäure, |
| 0,0240 | Gew.-% Furfurale, |
| 0,0005 | Gew.-% Allylacrylat, |
| 0,0017 | Gew.-% Allylformiat, |
| 0,0099 | Gew.-% Benzaldehyd, |
| 0,1591 | Gew.-% Maleinsäureanhydrid, |
| 0,0192 | Gew.-% Benzoesäure, |
| 0,0250 | Gew.-% Phthalsäureanhydrid, |
| 0,5083 | Gew.-% Diacrylsäure, |
| 1,0429 | Gew.-% Polyacrylsäure, |
| 0,0126 | Gew.-% Phenothiazin, |
| 0,0146 | Gew.-% MEHQ, |
| 0,1198 | Gew.-% sonstige hochsiedende Bestandteile, und |
| 0,0011 | Gew.-% Sauerstoff. |

In den Sumpfraum 5 der Kondensationskolonne 28 ist ein Zentrifugaltropfenabscheider integriert, der verhindert, dass Tröpfchen der Sumpfflüssigkeit aus dem Sumpfraum heraus nach oben mitgerissen werden. Am unteren Ende des Sumpfraums 5 ist ein sogenannter Chinesen-Hut zur verbesserten Gas/Flüssig-Trennung installiert. Die Flüssigphasenvorhaltemenge (der Flüssigkeitsholdup) im System aus Sumpfraum 5, Rohrleitung 6 zur Umlaufpumpe 7, Umlaufpumpe 7, Rohrleitung 6' von der Umlaufpumpe 7 zum Direktumlaufverdampfer 1, Direktumlaufverdampfer 1 und Rohrleitung 2 vom Direktumlaufverdampfer 1 in den Sumpfraum 5 beträgt 80 m$^3$.

Der Sumpfraum der Trennkolonne 28 ist, wie bereits erwähnt, auf einer Kolonnenhöhe (wie alle Höhen vom Sumpfboden aus gerechnet) von 7,80 m durch einen ersten Fangboden 10 (Sammelboden, Kaminboden mit 16 etwa gleichverteilten bedachten Kaminen; Kamindurchmesser : 600 mm; Kaminhöhe : 1 m) abgeschlossen.

Der Sammelboden 10 ist doppelwandig mit 2°Gefälle nach Innen und mit zentraler Abzugstasse und Abzugsstutzen (DN-200) gestaltet. Der freie Gasquerschnitt beträgt ca. 30%.

Von diesem ersten Fangboden werden, wie bereits erwähnt, 63009 kg/h Flüssigkeit entnommen (T = 100,4°C, p = 1,48 bar) und mittels der Kreiselpumpe 29 in den Direktumlaufverdampfer 1 geführt. Das Flüssigkeitsvolumen auf dem Fangboden 10 beträgt 2 m$^3$ (der Flüssigkeitsholdup im System aus diesem Flüssigkeitsvolumen auf dem Fangboden 10, Rohrleitung 3 zur Umlaufpumpe 29, Umlaufpumpe 29 und Rohrleitung 3' von der Umlaufpumpe 29 zum Direktumlaufverdampfer 1 beträgt 3 m$^3$).

2,0 m oberhalb des ersten Fangbodens 10 befindet sich der erste (11) von zunächst 15 Dual-Flow-Böden. Diese Dual-Flow-Böden (Lochdurchmesser einheitlich 14 mm, Lochanzahl einheitlich 33678, Öffnungsverhältnis einheitlich 18%) sind äquidistant angebracht mit einem Bodenabstand von 380 mm. Die Durchtrittsöffnungen bestehen aus kreisrunden Öffnungen des einheitlichen Durchmessers von 14 mm, wobei der Stanzgrat in der Trennkolonne nach unten zeigt. Die Anordnung der Mittelpunkte der Durchtrittskreise folgt einer strengen Dreiecksteilung.

Der fünfzehnte Dual-Flow-Boden (12) ist als Verteilerboden gestaltet. Zu diesem Zweck sind über ihm zwei Einsteckrohre (DN-150) mit 40 Auslaufbohrungen (Durchmesser: 15 mm) je Einsteckrohr angebracht.

Die erste Serie von Dual-Flow-Böden wird mit einem zweiten Fangboden 14 (Sammelboden; Kaminboden mit 16 ca. gleichmäßig verteilten bedachten Kaminen; Kaminhöhe ca. 1,70 m, zentrale Abzugstasse mit seitlichen Abzugsstutzen (DN-250), freier Gasquerschnitt von - 30 %) gemäß WO 03 047714 abgeschlossen, der 1,50 m oberhalb des letzten Dual-Flow-Bodens untergebracht ist.

Von diesem zweiten Fangboden 14 wird über die Leitung 15 bei 1,47 bar kontinuierlich rohe Acrylsäure mit einer Temperatur von 101,2˚C entnommen (Massendichte = 956,99 kg/m³), die folgende Gehalte aufweist:

| | |
|---|---|
| 96,8011 | Gew.-% Acrylsäure, |
| 0,4598 | Gew.-% Essigsäure, |
| 1,4762 | Gew.-% Wasser, |
| 0,0137 | Gew.-% Ameisensäure, |
| 0,0015 | Gew.-% Formaldehyd |
| 0,0087 | Gew.-% Acrolein, |
| 0,0647 | Gew.-% Propionsäure, |
| 0,2856 | Gew.-% Furfurale, |
| 0,0027 | Gew.-% Allylacrylat, |
| 0,0002 | Gew.-% Allylformiat, |
| 0,0744 | Gew.-% Benzaldehyd, |
| 0,2381 | Gew.-% Maleinsäureanhydrid, |
| 0,5430 | Gew.-% Diacrylsäure, |
| 0,0120 | Gew.-% Phenothiazin, |
| 0,0180 | Gew.-% MEHQ, und |
| 0,0004 | Gew.-% Sauerstoff. |

Das Flüssigkeitsvolumen auf dem zweiten Fangboden 14 beträgt 10 m³. 455855 kg/h der dem zweiten Fangboden 14 entnommenen rohen Acrylsäure wird durch indirekten Wärmetausch auf 111,2˚C erwärmt und über die Leitung 30 unmittelbar unterhalb des dem zweiten Fangboden nach oben folgenden Dual-Flow-Bodens 16 in die Kondensationskolonne 28 rückgeführt (p = 1,50 bar).

89978 kg/h der dem zweiten Fangboden entnommenen rohen Acrylsäure bilden den im trennwirksamen Raum abgetrennten, (Meth)acrylmonomere enthaltenden Stoffstrom und werden mehrstufig durch indirekten Wärmetausch (bevorzugt wärmeintegriert gegen in die Trennkolonne 28 rückzuführende Mutterlauge) auf eine Temperatur von 29˚C abgekühlt. Dann werden der abgekühlten rohen Acrylsäure 1144 kg/h Wasser (25˚C) zugefügt. Das resultierende Gemisch wird durch nochmaligen indirekten Wärmeaustausch auf 20˚C abgekühlt und dann in zwei bis drei Kühlscheibenkristallisatoren geführt.

Dabei handelt es sich jeweils um einen Trog, in welchem 20 bis 24 gewischte kreisförmige Kühlplatten (die innerlich von einem Kühlmedium (Gemisch aus Wasser und Glykol; Glykolanteil = 10 bis 50 Gew.-%, bevorzugt 25 bis 35 Gew.-%) durchflossen werden) im äquidistanten Abstand von 20 bis 40 cm hintereinander hängend angeordnet sind (Plattendurchmesser typisch 2 bis 4 m, bevorzugt 2,5 bis 3 m). Das Kühlmedium wird dabei im Gegenstrom zum kristallisierenden Gemisch durch den Kristallisator von Kühlscheibe zu Kühlscheibe weitergereicht. Es kann aber auch aufgeteilt auf 2 bis 3 parallele Ströme über die Kühlplatten geführt werden. Die Eintrittstemperatur des Kühlmediums (der Sole) beträgt -2 bis +5˚C. Die Austrittstemperatur liegt 2 bis 7˚C höher. Durch das Wischen der Kühlplatten wird die Ausbildung einer Kristallschicht unterdrückt. Die angewässerte rohe Acrylsäure wird von hinten nach vorne kontinuierlich durch den Kristaller geführt (gepumpt oder überlaufgeregelt). Die einphasige angewässerte rohe Acrylsäure verdickt dabei (Verweilzeit 0,5 bis 4 h, bevorzugt 1,5 bis 2,5 h) zu einer zweiphasigen, Acrylsäurekristalle als feste Phase enthaltenden Suspension einer Temperatur von 6 bis 11˚C und einem Feststoffgehalt am Austritt von 20 bis 35 Gew.-%. Die Drehzahl der Wischer beträgt 2 bis 15 Umdrehungen je Minute, bevorzugt 4 bis 10 Umdrehungen je Minute. Die die Wischer treibende, zentriert durch die Kühlscheiben geführte Welle, ist mit wassergespülten Stopfbuchspackungen (Packungsschnüre aus Teflon oder Graphit) abgedichtet.

Auf dem Umfang der Kühlscheiben, wo nicht gewischt werden kann, ist ein Hohlprofil (z.B. in einfachster Ausführungsform ein Rohr) aufgebracht (z.B. aufgeschweißt), das mittels eines zweiten Wärmeträgers (z.B. ebenfalls Wasser/Glykol Gemisch) beheizt wird (auf eine Temperatur oberhalb der Kristallisationstemperatur; meist aus dem Temperaturbereich 8 bis 20˚C, bevorzugt 10 bis 14˚C). Diese Umfangbeheizungen werden mit dem zweiten Wärmeträger parallel angeströmt.

Darüber hinaus sind die Wischer in radialer Richtung bevorzugt segmentiert (≥2, ≤6 Segmente in der Regel). Die spezifische Anpresskraft der Wischer liegt im eingebauten Zustand senkrecht zur Kühlfläche bei 1 bis 10, bevorzugt 3 bis 5 N pro cm aktiver Wischkantenlänge. Zusätzlich zu den Wischern treibt die Welle Paddel an (zwischen zwei Kühlscheiben und vor der ersten und letzten Kühlscheibe zweckmäßig jeweils zwei in symmetrischer Anordnung), die eine verbesserte Durchmischung bewirken.

Die Beschaffenheit der Kühlplattenoberfläche und die Ausrichtung der Wischer ist so, dass der Abstand der Wischer zur Kühlplattenoberfläche an keinem Punkt 6 mm überschreitet (günstig ist ein Betrieb bei dem der vorgenannte Abstand an keinem Punkt 4 bzw. 2 oder 1 mm überschreitet, bzw. bei dem die Wischer an jedem Punkt anliegen; als besonders vorteilhaft erweist sich ein Anliegen am äußeren Radius;).

Im in Förderrichtung der Suspension hinteren Teil des Kristallisators (bevorzugt hinter der letzten Kühlscheibe) wird die Suspension über ein angeschlossenes Rohr (zweckmäßigerweise getaucht angebracht; alternativ kann die Suspension über ein Überlaufwehr in einen gerührten Sammelbehälter fließen, von dem aus die Waschkolonnen beschickt werden) zu hydraulischen Schmelze-Waschkolonnen geführt, wie sie in der DE-A 10 156 016 und in der DE-A 10 223 058 beschrieben sind, um die Mutterlauge vom Suspensionskristallisat abzutrennen. Die Beschickung der Waschkolonnen mit Kristallsuspension erfolgt mittels einer Kreisel- oder Kreiskolbenpumpe. Die Steuerstrompumpe ist ebenfalls als Kreiskolbenpumpe oder als Kreiselpumpe mit Regelventil ausgeführt. Der Druck am unteren Ende einer Waschkolonne ist üblicherweise ≥ 100 mbar und ≤5 bar kleiner als der Druck am Kopf der Waschkolonne. Der Kopfdruck beträgt in der Regel bis zu 6 bar, meist 0,5 bis 4 bar. Die Messerdrehzahl liegt meist bei Werten > 0 und ≤100/min, bzw. ≤60/min. Die Temperatur im Schmelzkreis beträgt normal 13 bis 16°C. Die Erfassung der Filtrationsfront wird gemäß der DE-A 10 036 880 über 2 bis 4 optische Sensoren vorgenommen. Die Waschfront wird mittels Temperaturmessung im Kristallbett geregelt.

Die Gesamthöhe des Kristallbetts liegt typisch bei 300 bis 1500 mm, meist bei 400 bis 1000 mm. Die Waschfront befindet sich typisch 10 bis 400 mm, meist bei 20 bis 250 mm oberhalb des Messers. Als Schmelzkreispumpe eignet sich eine Kreiselpumpe mit produktseitiger Spülung der Wellenabdichtung (Gleitringdichtung) oder eine magnetgekuppelte Pumpe mit erhöhter Gleitlagerspülung. Die Umlaufmenge im Schmelzkreis beträgt 2 bis 30, meist 5 bis 20 m$^3$/h pro Tonne mit dem Messer abgetragenem gereinigtem Kristallisat. Die Stabilisierung des Schmelzkreises erfolgt mittels 100 bis 300 Gew.-ppm MEHQ. Zusätzlich wird in den Schmelzkreis Luft eingetragen, deren Überschuß (= nicht in der Waschschmelze gelöster Anteil) vor Eintritt der Waschschmelze in die Waschkolonne via Gasabscheider abgetrennt wird.

[a] Zur Herstellung einer veresterungsgerechten Acrylsäure ist es ausreichend die Abtrennung des Suspensionskristallisats anstelle in einer Schmelze-Waschkolonne mittels einer Zentrifuge (z-B. einer 2- oder 3-stufigen Schubzentrifuge) durchzuführen. Geeignete Siebspaltweiten liegen bei 150 bis 300 mm; anwendbare Zentrifugalbeschleunigungen liegen bei 500 bis 900 g, meist 600 bis 800 g; geeignet sind Hubzahlen von 40 bis 80 Schüben/min. Vorzugsweise werden die auf der 2. oder 3. Stufe der Zentrifuge abgetrennten Kristalle mit 0,15 bis 0,3 kg Waschflüssigkeit pro kg Kristallisat gewaschen. Die Temperatur der Waschflüssigkeit liegt bei 15 bis 30°C, bevorzugt bei 20 bis 30°C. Zur Vermeidung von Ablagerungen wird der Feststoffabwurfschacht der Zentrifuge mit auf 15 bis 30°C temperierter Spülflüssigkeit gespült. Spül- und Waschflüssigkeit sind bevorzugt aufgeschmolzenes, über die Zentrifuge abgetrenntes und gewaschenes Kristallisat. Zur Vermeidung von Ablagerungen und Verkrustungen ist es zweckmäßig, das Zentrifugengehäuse, das Suspensionszuführrohr und das Waschflüssigkeitszuführrohr auf eine Temperatur ≥15°C und ≤40°C zu temperieren. Der Produktraum der Zentrifuge wird zweckmäßig mit Stickstoff oder mit einem Gemisch aus Luft und Stickstoff inertisiert. Die Wellenabdichtung wird mit Gas (z.B. Stickstoff oder ein Gemisch aus Luft und Stickstoff) oder mit Wasser gespült.

(b) Alternativ zur Suspensionskristallisation kann auch eine Schichtkristallisation (z.B. Fallfilmkristallisation gemäß EP-A 616 998 oder voll durchströmtes Rohr) mit 3 oder mehr (z.B. 3 bis 4) Reinigungsstufen angewendet werden. Anstelle die Mutterlauge einer nachgehenden Reinigungsstufe in eine vorgehende Reinigungsstufe rückzuführen, kann man sie auch gemeinsam in die Kondensationskolonne rückführen.]

Den Schmelzkreisen, die durch den Zusatz von 3 kg/h MEHQ stabilisiert werden, werden 18538 kg/h an Reinacrylsäure der nachfolgende Gehalte entnommen:

| | |
|---|---|
| 99,8335 | Gew.-% Acrylsäure, |
| 0,0970 | Gew.-% Essigsäure, |
| 0,0334 | Gew.-% Wasser, |
| 0,0206 | Gew.-% Propionsäure, |
| 0,0001 | Gew.-% Furfurale, |
| 0,0001 | Gew.-% Maleinsäureanhydrid, |
| 0,0003 | Gew.-% Diacrylsäure, und |
| 0,0150 | Gew.-% MEHQ. |

Sie eignet sich in hervorragender Weise zur Herstellung von Superabsorbern auf der Basis von Poly-Na-Acrylat.

In 829 kg/h der Reinacrylsäure werden 13 kg/h PTZ zur Herstellung einer Inhibitorlösung I. gelöst. In 30 kg/h Inhibitorlösung I werden 19 kg/h MEHQ unter Bildung der Inhibitorlösung II gelöst.

Die in den Waschkolonnen abgetrennte Mutterlauge wird zunächst in einen heizbaren Sammelbehälter und von dort in einen Tank gefahren. Von diesem wird sie wärmeintegriert auf 90°C erwärmt in einer Menge von 71759 kg/h über die Leitung 13 auf den fünfzehnten Dual-Flow-Boden 12 der Kondensationskolonne 28 (von unten gerechnet) rückgeführt. Die Gehalte dieser rückgeführten Mutterlauge sind wie folgt:

|  |  |
|---|---|
| 94,4349 | Gew.-% Acrylsäure, |
| 0,5504 | Gew.-% Essigsäure, |
| 3,4362 | Gew.-% Wasser, |
| 0,0172 | Gew.-% Ameisensäure, |
| 0,0018 | Gew.-% Formaldehyd, |
| 0,0109 | Gew.-% Acrolein, |
| 0,0756 | Gew.-% Propionsäure, |
| 0,3580 | Gew.-% Furfurale, |
| 0,0034 | Gew.-% Allylacrylat, |
| 0,0003 | Gew.-% Allylformiat, |
| 0,0933 | Gew.-% Benzaldehyd, |
| 0,2986 | Gew.-% Maleinsäureanhydrid, |
| 0,6808 | Gew.-% Diacrylsäure, |
| 0,0150 | Gew.-% Phenothiazin, |
| 0,0233 | Gew.-% MEHQ, und |
| 0,0005 | Gew.-% Sauerstoff. |

Zusätzlich werden auf den Dual-Flow-Boden 12 15224 kg/h an vom Sammelboden 14 entnommener roher Acrylsäure rückgeführt (32).

2,9 m oberhalb des zweiten Fangbodens 14 befindet sich in der Kondensationskolonne 28 der erste (16) von 21 weiteren Dual-Flow-Böden der bereits beschriebenen Art (Lochdurchmesser wieder einheitlich 14 mm, Lochanzahl jedoch einheitlich 32020 und Öffnungsverhältnis einheitlich 17,4 %), die wieder äquidistant mit einem Bodenabstand von 380 mm angeordnet sind. Der Rücklauf aus dem Thormann-Boden-Bereich in den Dual-Flow-Boden Bereich erfolgt über ein in die Kolonne integriertes Verteilersystem. Alternativ könnte er auch durch unterhalb des untersten Thormann-Bodens erfolgendes Herausführen der Rücklaufflüssigkeit aus der Kondensationskolonne mittels einer Pumpe und Rückführen in die Kondensationskolonne über zwei (oder mehrere) Einsteckrohre erfolgen, die oberhalb des obersten Dual-Flow-Bodens angebracht sind.

800 mm oberhalb des letzten Dual-Flow-Bodens beginnt sich die Kondensatioriskolonne konisch zu erweitern. 500 mm oberhalb des letzten Dual-Flow-Bodens endet diese Erweiterung bei einem Kolonneninnendurchmesser von 6,50 m.

Auf dieser Höhe, d.h., 1,50 m oberhalb des letzten Dual-Flow-Bodens (17), beginnt eine äquidistante (Bodenabstand = 500 mm) Anordnung von 28 konventionellen, einflutigen Thormann-Böden. Die Thormann-Böden sind derart ausgestaltet, dass über die Anordnung der Treibschlitze in den Hauben der Thormann-Böden in in Querstrom-Richtung aufeinanderfolgenden Rinnen jeweils eine zueinander entgegengesetzte Strömungsrichtung der Flüssigkeit erzeugt wird.

Das Öffnungsverhältnis der Thormann-Böden beträgt 14 %. Das Verhältnis von Kaminfläche zu Schlitzaustrittsfläche beträgt 0,8. Die Kaminhöhe und die Höhe des Ablaufwehrs beträgt 40 mm. Die Bodenfreiheit der Glocke (Abstand zwischen Unterkante Schlitz und Boden) beträgt 10 mm. Die Schlitzhöhe beträgt 15 mm. Der Winkel zwischen ausgestelltem Schlitz und Längskante der Haube beträgt 30 Grad. Die Länge der Längskante der Haube beträgt maximal 800 mm. Im Randbereich der Kolonne reduziert sich die Haubenlänge auf bis zu 200 mm aus Gründen der Anpassung an die Rundheit der Kolonne. Der Abstand zwischen zwei in Querstromrichtung auf einer Linie befindlichen Hauben beträgt 66 mm. Die Ablauffläche des Ablaufschachts beträgt 1,5 % bezogen auf die Querschnittsfläche des Bodens. Die Breite zwischen den beiden unteren Längsrändern einer Haube beträgt 64 mm.

Auf der Höhe des obersten Thormann-Bodens 20 beginnt sich die Trennkolonne wieder konisch zu verengen. 700 mm oberhalb des obersten Thormann-Bodens ist diese Verengung abgeschlossen und der Kolonneninnendurchmesser wieder auf 6,00 m zurückgeschrumpft.

1,70 m oberhalb des obersten Thormann-Bodens befindet sich der dritte Fangboden 22 (Sammelboden, Kaminboden mit 16 ca. gleichmäßig verteilten bedachten Kaminen, Kaminhöhe = 1,50 m). Das Flüssigkeitsvolumen auf dem Fangboden 3 beträgt 8 m$^3$ (Massendichte = 964,38 kg/m$^3$).

Vom dritten Fangboden werden 533617 kg/h Sauerwasser mit einer Temperatur von 68,6°C und bei einem Druck von 1,24 bar über die Leitung 23 entnommen.

Die Gehalte des Sauerwassers sind:

| | |
|---|---|
| 11,3387 | Gew.-% Acrylsäure, |
| 4,1574 | Gew.-% Essigsäure, |
| 81,6277 | Gew.-% Wasser, |
| 0,5256 | Gew.-% Ameisensäure, |
| 2,3082 | Gew.-% Formaldehyd, |
| 0,0154 | Gew.-% Acrolein, |
| 0,0089 | Gew.-% Propionsäure, |
| 0,0024 | Gew.-% Furfurale, |
| 0,0135 | Gew.-% Allylformiat, |
| 0,0001 | Gew.-% MEHQ, und |
| 0,0021 | Gew.-% Sauerstoff. |

29015 kg/h des entnommenen Sauerwassers (68,6°C) werden (21) zusammen mit der Inhibitorlösung II (31) auf den obersten Thormann-Boden (20) rückgeführt.

812 kg/h der Inhibitorlösung I werden (von unten betrachtet) auf den 19ten Thormann-Boden (18) rückgeführt (mit einer Temperatur von 25°C und einem Druck von 3 bar über die Leitung 19).

7282 kg/h des entnommenen Sauerwassers werden der Verbrennung zugeführt.

298392 kg /h des entnommenen Sauerwassers werden über die Leitung 25 mit einer Temperatur von 29°C auf den sechsten (24) der nachfolgend zu beschreibenden Ventilböden (von unten gerechnet) rückgeführt (3 bar). Im Fall von Schaumbildung erweist sich die Zugabe von Entschäumer (z.B. alkoxylierte Alkohole wie Dekresa® SD 23 oder Mischungen aus Fettsäure, Polylglykol, Emulgatoren und paraffinischem Mineralölgemisch wie Nalco® 71-D-5) auf den dritten Fangboden (z.B. in einer Menge von 30 ml/h) als zweckmäßig.

198928 kg/h des entnommenen Sauerwassers werden über die Leitung 27 mit einer Temperatur von 22,5°C (p=3 bar) auf den obersten (26) der nachfolgend zu beschreibenden Ventilböden rückgeführt.

2300 mm oberhalb des dritten Fangbodens 22 sind in der Kondensationskolonne 28 in äquidistanter Anordnung (Bodenabstand = 500 mm) 11 zweiflutige Ventilböden angebracht. Die Höhe des Ablaufwehrs beträgt 35 mm. Das Öffnungsverhältnis liegt bei 18 % und die Summe der Ablaufflächen der Ablaufschächte von zwei aufeinanderfolgenden Ventilböden beträgt 10 % der Kolonnenquerschnittsfläche. Als Ventile wurden VV12-Ventile der Fa. Stahl, DE, Viernheim verwendet.

Der Druck am Kopf der Kolonne 28 beträgt 1,2 bar.

Am Kolonnenkopf verlassen 164650 kg/h Abgas (33) mit einer Temperatur von 33,5°C und den nachfolgenden Gehalten über einen Demister die Trennkolonne 28:

| | |
|---|---|
| 0,2288 | Gew.-% Acrylsäure, |
| 0,0885 | Gew.-% Essigsäure, |
| 2,6689 | Gew.-% Wasser, |
| 0,0052 | Gew.-% Ameisensäure, |
| 0,1724 | Gew.-% Acrolein, |
| 0,0002 | Gew.-% Propionsäure, |
| 0,0003 | Gew.-% Furfurale, |
| 0,0012 | Gew.-% Allylformiat, |
| 2,1235 | Gew.-% $CO_2$, |
| 0,6939 | Gew.-% CO, |
| 0,6487 | Gew.-% Propan, |
| 0,3169 | Gew.-% Propylen, |
| 4,7392 | Gew.-% $O_2$, und |
| 88,3123 | Gew.-% $N_2$. |

In einem indirekten Wärmetauscher wird das Abgas auf 38°C erwärmt und anschließend werden 91196 kg/h dieses Abgases über einen Kreisgasverdichter (z.B. ein Radialverdichter) als Verdünnungsgas in die Gasphasenoxidation und in die Rückspaltung geführt und 73455 kg/h des Abgases werden der Verbrennung zugeführt.

Insgesamt werden in den trennwirksamen Raum damit folgende Einzelströme hineingeführt, die die folgenden Acrylsäuregehalte aufweisen:

170008 kg/h Produktgasgemisch der Gasphasenpartialoxidation von Propylen mit einem Acrylsäuregehalt von 11,8 Gew.-%;

21883 kg/h über die Leitung 9, mit einem Acrylsäuregehalt von 8,7215 Gew.-%;
15224 kg/h über die Leitung 32, mit einem Acrylsäuregehalt von 96,8011 Gew.-%;
71759 kg/h über die Leitung 13, mit einem Acrylsäuregehalt von 94,4349 Gew.-%;
455855 kg/h über die Leitung 30, mit einem Acrylsäuregehalt von 96,7887 Gew.-%;
81,2 kg/h über die Leitung 19, mit einem Acrylsäuregehalt von 98,3360 Gew.-%;
29015 kg/h über die Leitung 21, mit einem Acrylsäuregehalt von 11,3387 Gew.-%:
und 49 kg/h über die Leitung 31, mit einem Acrylsäuregehalt von 60,2057 Gew.-%.

Damit wird in den trennwirksamen Raum insgesamt ein (gedanklich erzeugter) Stoffstrom von 764605 kg/h hineingeführt, dessen Acrylsäuregehalt 71,907 Gew.-% beträgt. Damit beträgt X = 28,09 Gew.-%.

Der Stoffstrom, der mit dem höchsten Gewichtsanteil an Acrylsäure aus dem trennwirksamen Raum herausgeführt wird, ist die vom zweiten Sammelboden weggeführte rohe Acrylsäure, mit einem Acrylsäuregehalt von 96,8011 Gew.-% Acrylsäure. Damit beträgt Y = 3,20 Gew.-% und X : Y = 8,78.

Das im trennwirksamen Raum mit Flüssigphase gefüllte Gesamtvolumen beträgt 167,5 $m^3$. Die Temperatur dieser Flüssigphase beträgt wenigstens teilweise 120,2°C (z.B. im Direktumlaufwärmetauscher).

Die nachfolgende Tabelle 1 zeigt die gewählten Volumenelemente i ("Boden i" steht dabei für das Volumenelement i, das die auf dem Stoffaustauschboden i befindliche Flüssigphase und die im Volumen unterhalb des Stoffaustauschbodens i befindliche Flüssigphase (ausnehmlich der auf dem darunter liegenden Boden befindlichen Flüssigphase) erfasst). Die Nummerierung der Stoffaustauschböden nummeriert in der Trennkolone von untern nach oben.

Ferner enthält die Tabelle 1 die ermittelten Temperaturen $T_i$, die ermittelten Werte $m_{si}$ und die ermittelten Massenströme $\dot{m}_i$.

Auch enthält die Tabelle 1 die individuellen Werte $t_i^* = 2^A m_{si}/\dot{m}_i$.

Tabelle 1

| Volumenelement i | $m_{si}$ (kg) | $\dot{m}_i$ (kg/h) | $t_i^*$ (h) | $T_i$ (°C) |
|---|---|---|---|---|
| Gesamtvolumen aus Rohrleitungen 6, 6' und 2 sowie Pumpe 7, Sumpfraum 5 und Direktumlaufverdampfer 1 | 21828,8 | 2300,0 | 33,74320 | 118,3 |
| Sammelboden 1 einschließlich Rohrleitungen 3 und 3' | 2663,6 | 63009,2 | 0,043353 | 100,4 |
| Boden 1 | 301,9 | 63009,2 | 0,004913 | 100,4 |
| Boden 2 | 303,7 | 68968,9 | 0,004456 | 100,2 |
| Boden 3 | 305,6 | 74928,7 | 0,004072 | 100,0 |
| Boden 4 | 307,5 | 80888,4 | 0,003744 | 99,8 |
| Boden 5 | 309,3 | 86848,2 | 0,003461 | 99,6 |
| Boden 6 | 311,1 | 92807,9 | 0,003214 | 99,4 |
| Boden 7 | 311,6 | 92865,4 | 0,003123 | 99,0 |
| Boden 8 | 312,2 | 92923,0 | 0,003035 | 98,5 |
| Boden 9 | 312,7 | 92980,5 | 0,002949 | 98,1 |
| Boden 10 | 313,2 | 93038,1 | 0,002866 | 97,7 |
| Boden 11 | 313,7 | 96839,1 | 0,002873 | 98,3 |
| Boden 12 | 314,1 | 100640,1 | 0,002885 | 98,9 |
| Boden 13 | 314,6 | 104441,1 | 0,002901 | 99,5 |
| Boden 14 | 315,0 | 108242,1 | 0,002920 | 100,0 |
| Boden 15 | 315,5 | 112043,1 | 0,002944 | 100,6 |
| Sammelboden 2 | 9292,9 | 115844,1 | 0,087376 | 101,2 |
| Boden 16 | 265,8 | 115844,1 | 0,002499 | 101,2 |

(fortgesetzt)

| Volumenelement i | $m_{si}$ (kg) | $\dot{m}_i$ (kg/h) | $t_i^*$ (h) | $T_i$ (°C) |
|---|---|---|---|---|
| Boden 17 | 266,1 | 118186,3 | 0,002441 | 101,2 |
| Boden 18 | 266,3 | 120528,5 | 0,002385 | 101,1 |
| Boden 19 | 266,6 | 122870,7 | 0,002332 | 101,0 |
| Boden 20 | 266,9 | 125212,9 | 0,002280 | 101,0 |
| Boden 21 | 267,2 | 127555,1 | 0,002230 | 100,9 |
| Boden 22 | 267,2 | 127588,1 | 0,002223 | 100,9 |
| Boden 23 | 267,2 | 127621,0 | 0,002215 | 100,8 |
| Boden 24 | 267,2 | 127654,0 | 0,002207 | 100,8 |
| Boden 25 | 267,3 | 127686,9 | 0,002199 | 100,7 |
| Boden 26 | 267,3 | 127719,9 | 0,002192 | 100,7 |
| Boden 27 | 267,3 | 127752,9 | 0,002184 | 100,6 |
| Boden 28 | 267,3 | 127785,8 | 0,002176 | 100,6 |
| Boden 29 | 267,3 | 127760,1 | 0,002165 | 100,5 |
| Boden 30 | 267,3 | 127734,3 | 0,002154 | 100,4 |
| Boden 31 | 267,3 | 127708,5 | 0,002144 | 100,3 |
| Boden 32 | 267,3 | 127682,8 | 0,002133 | 100,3 |
| Boden 33 | 267,3 | 127657,0 | 0,002122 | 100,2 |
| Boden 34 | 267,2 | 127631,3 | 0,002111 | 100,1 |
| Boden 35 | 267,2 | 127605,5 | 0,002101 | 100,0 |
| Boden 36 | 267,1 | 127381,4 | 0,002065 | 99,8 |
| Boden 37 | 1428,6 | 127157,2 | 0,010862 | 99,5 |
| Boden 38 | 1428,1 | 126933,1 | 0,010679 | 99,2 |
| Boden 39 | 1427,6 | 126709,0 | 0,010498 | 99,0 |
| Boden 40 | 1422,6 | 125231,0 | 0,009699 | 97,7 |
| Boden 41 | 1417,4 | 123752,9 | 0,008960 | 96,5 |
| Boden 42 | 1371,5 | 115844,1 | 0,006492 | 91,3 |
| Boden 43 | 1304,4 | 107584,7 | 0,005208 | 87,8 |
| Boden 44 | 1236,7 | 99325,3 | 0,004190 | 84,3 |
| Boden 45 | 994,1 | 77092,8 | 0,003002 | 79,0 |
| Boden 46 | 869,1 | 67710,3 | 0,002705 | 77,5 |
| Boden 47 | 744,5 | 58327,8 | 0,002436 | 76,1 |
| Boden 48 | 684,1 | 54345,3 | 0,002313 | 75,6 |
| Boden 49 | 624,0 | 50362,8 | 0,002191 | 75,0 |
| Boden 50 | 357,7 | 46380,3 | 0,001313 | 74,5 |
| Boden 51 | 328,9 | 43818,9 | 0,001247 | 74,1 |
| Boden 52 | 300,3 | 41257,4 | 0,001180 | 73,7 |
| Boden 53 | 271,8 | 38696,0 | 0,001111 | 73,4 |
| Boden 54 | 253,0 | 37314,8 | 0,001054 | 73,1 |

(fortgesetzt)

| Volumenelement i | $m_{si}$ (kg) | $\dot{m}_i$ (kg/h) | $t_i^*$ (h) | $T_i$ (°C) |
|---|---|---|---|---|
| Boden 55 | 234,3 | 35933,6 | 0,000997 | 72,9 |
| Boden 56 | 215,7 | 34552,4 | 0,000938 | 72,7 |
| Boden 57 | 202,2 | 33699,1 | 0,000890 | 72,5 |
| Boden 58 | 188,8 | 32845,8 | 0,000841 | 72,3 |
| Boden 59 | 175,3 | 31992,5 | 0,000791 | 72,1 |
| Boden 60 | 164,2 | 31391,2 | 0,000744 | 71,9 |
| Boden 61 | 153,1 | 30789,8 | 0,000696 | 71,6 |
| Boden 62 | 142,0 | 30188,4 | 0,000648 | 71,4 |
| Boden 63 | 124,3 | 29626,2 | 0,000524 | 70,0 |
| Boden 64 | 106,6 | 29064,0 | 0,000416 | 68,6 |
| Sammelboden 3 | 889,1 | 533617,0 | 0,000189 | 68,6 |
| Boden 65 | 222,3 | 533617,0 | 0,000047 | 68,6 |
| Boden 66 | 221,7 | 529532,3 | 0,000038 | 65,2 |
| Boden 67 | 221,1 | 525447,6 | 0,000030 | 61,8 |
| Boden 68 | 222,0 | 520416,7 | 0,000020 | 55,9 |
| Boden 69 | 222,9 | 515385,9 | 0,000014 | 50,0 |
| Boden 70 | 223,4 | 504553,3 | 0,000013 | 48,6 |
| Boden 71 | 201,5 | 204568,8 | 0,000025 | 47,2 |
| Boden 72 | 202,2 | 203298,7 | 0,000021 | 44,6 |
| Boden 73 | 202,8 | 202028,6 | 0,000018 | 42,0 |
| Boden 74 | 203,4 | 200478,3 | 0,000014 | 37,8 |
| Boden 75 | 204,1 | 198928,0 | 0,000010 | 33,5 |

Damit ergibt sich ein $t_{ges}$ von 34,06 h.

Bei diesem $t_{ges}$ werden als im trennwirksamen Raum abgetrennter (Meth)acrylmonomere enthaltender Stoffstrom mit dem höchsten Gewichtsanteil an Acrylsäure 89978 kg/h rohe Acrylsäure mit einem Acrylsäuregehalt von 96,8011 Gew.-% abgetrennt.

Beispiel (beschrieben wird der stationäre Zustand)

Es wird im wesentlichen alles wie im Vergleichsbeispiel durchgeführt, das Volumen des Sumpfraum 5 wird jedoch soweit verringert, das der Flüssigkeitsholdup im Sumpfraum 5 59974,2 kg weniger als im Vergleichsbeispiel beträgt (der Flüssigkeitsholdup im Volumenelement i bestehend aus den Rohrleitungen 6, 6' und 2 sowie aus Pumpe 7, Sumpfraum 5 und Direktumlaufverdampfer 1 beträgt somit im Vergleichsbeispiel 79965,6 kg und im Beispiel 19991,4 kg).

Außerdem beträgt die vom ersten Sammelboden 10 abgezogene und dem Direktumlaufverdampfer 1 zugeführte Menge an Schwersiedefraktion 74083 kg/h anstelle von 63009 kg/h.

Diese Änderung ist erforderlich um die im Sumpfraum 5 aufrund der veränderten Zusammensetzung der Sumpfflüssigkeit sich im Sumpfraum verringernde Siedetemperatur im Direktumlaufverdampfer 1 zu erreichen.

Die nachfolgende Tabelle 2 ist das Analogon zur Tabelle 1 für das Beispiel.

Tabelle 2

| Volumenelement i | $m_{si}$ (kg) | $\dot{m}_i$ (kg/h) | $t_i^*$ (h) | $T_i$ (°C) |
|---|---|---|---|---|
| Gesamtvolumen aus Rohrlei tungen 6, 6' und 2 sowie Pumpe 7, Sumpfraum 5 und Direktumlaufverdampfer 1 | 8780,0 | 2300,0 | 8,47136 | 111,5 |
| Sammelboden 1 einschließlich Rohrleitungen 3 und 3' | 2696,2 | 74083,0 | 0,03711 | 100,3 |
| Boden 1 | 305,6 | 74083,0 | 0,00421 | 100,3 |
| Boden 2 | 307,0 | 77918,5 | 0,00397 | 100,1 |
| Boden 3 | 308,3 | 81754,1 | 0,00375 | 99,9 |
| Boden 4 | 309,7 | 85589,6 | 0,00356 | 99,8 |
| Boden 5 | 311,1 | 89425,2 | 0,00338 | 99,6 |
| Boden 6 | 312,4 | 93260,7 | 0,00322 | 99,4 |
| Boden 7 | 312,8 | 93255,4 | 0,00313 | 99,0 |
| Boden 8 | 313,2 | 93250,0 | 0,00304 | 98,6 |
| Boden 9 | 313,6 | 93244,7 | 0,00295 | 98,1 |
| Boden 10 | 314,0 | 93239,3 | 0,00287 | 97,7 |
| Boden 11 | 314,4 | 97316,8 | 0,00287 | 98,3 |
| Boden 12 | 314,8 | 101394,3 | 0,00288 | 98,9 |
| Boden 13 | 315,2 | 105471,8 | 0,00288 | 99,5 |
| Boden 14 | 315,6 | 109549,3 | 0,00290 | 100,1 |
| Boden 15 | 316,0 | 113626,8 | 0,00291 | 100,7 |
| Sammelboden 2 | 9305,6 | 117704,3 | 0,08632 | 101,3 |
| Boden 16 | 266,1 | 117704,3 | 0,00247 | 101,3 |
| Boden 17 | 266,4 | 119728,4 | 0,00242 | 101,2 |
| Boden 18 | 266,7 | 121752,6 | 0,00237 | 101,1 |
| Boden 19 | 266,9 | 123776,7 | 0,00232 | 101,1 |
| Boden 20 | 267,2 | 125800,8 | 0,00228 | 101,0 |
| Boden 21 | 267,4 | 127825,0 | 0,00223 | 101,0 |
| Boden 22 | 267,5 | 127857,5 | 0,00223 | 100,9 |
| Boden 23 | 267,5 | 127890,0 | 0,00222 | 100,9 |
| Boden 24 | 267,5 | 127922,6 | 0,00221 | 100,8 |
| Boden 25 | 267,5 | 127955,1 | 0,00221 | 100,8 |
| Boden 26 | 267,5 | 127987,6 | 0,00220 | 100,7 |
| Boden 27 | 267,5 | 128020,1 | 0,00219 | 100,7 |
| Boden 28 | 267,5 | 128052,7 | 0,00218 | 100,6 |
| Boden 29 | 267,5 | 128032,7 | 0,00217 | 100,6 |
| Boden 30 | 267,5 | 128012,7 | 0,00216 | 100,5 |
| Boden 31 | 267,5 | 127992,7 | 0,00215 | 100,4 |
| Boden 32 | 267,5 | 127972,7 | 0,00214 | 100,4 |
| Boden 33 | 267,5 | 127952,7 | 0,00213 | 100,3 |
| Boden 34 | 267,5 | 127932,8 | 0,00212 | 100,2 |

(fortgesetzt)

| Volumenelement i | $m_{si}$ (kg) | $\dot{m}_i$ (kg/h) | $t_i^*$ (h) | $T_i$ (°C) |
|---|---|---|---|---|
| Boden 35 | 267,5 | 127912,8 | 0,00211 | 100,2 |
| Boden 36 | 267,5 | 127726,1 | 0,00208 | 99,9 |
| Boden 37 | 1430,9 | 127539,4 | 0,01098 | 99,7 |
| Boden 38 | 1430,7 | 127352,6 | 0,01081 | 99,4 |
| Boden 39 | 1430,5 | 127165,9 | 0,01065 | 99,2 |
| Boden 40 | 1427,2 | 125904,8 | 0,00993 | 98,1 |
| Boden 41 | 1423,7 | 124643,7 | 0,00926 | 97,0 |
| Boden 42 | 1386,2 | 117704,3 | 0,00689 | 92,3 |
| Boden 43 | 1327,1 | 110026,5 | 0,00553 | 88,8 |
| Boden 44 | 1267,4 | 102348,7 | 0,00445 | 85,2 |
| Boden 45 | 1035,0 | 80031,0 | 0,00311 | 79,4 |
| Boden 46 | 905,3 | 70048,9 | 0,00279 | 77,9 |
| Boden 47 | 775,9 | 60066,8 | 0,00250 | 76,3 |
| Boden 48 | 711,2 | 55777,7 | 0,00237 | 75,7 |
| Boden 49 | 646,8 | 51488,6 | 0,00224 | 75,1 |
| Boden 50 | 369,4 | 47199,5 | 0,00134 | 74,5 |
| Boden 51 | 339,0 | 44494,6 | 0,00127 | 74,2 |
| Boden 52 | 308,8 | 41789,7 | 0,00120 | 73,8 |
| Boden 53 | 278,6 | 39084,7 | 0,00113 | 73,4 |
| Boden 54 | 259,0 | 37647,5 | 0,00107 | 73,2 |
| Boden 55 | 239,5 | 36210,2 | 0,00101 | 72,9 |
| Boden 56 | 220,0 | 34773,0 | 0,00095 | 72,7 |
| Boden 57 | 206,0 | 33895,4 | 0,00090 | 72,5 |
| Boden 58 | 192,1 | 33017,8 | 0,00085 | 72,3 |
| Boden 59 | 178,3 | 32140,2 | 0,00080 | 72,1 |
| Boden 60 | 166,9 | 31526,3 | 0,00075 | 71,9 |
| Boden 61 | 155,5 | 30912,4 | 0,00071 | 71,7 |
| Boden 62 | 144,2 | 30298,5 | 0,00066 | 71,4 |
| Boden 63 | 126,1 | 29725,8 | 0,00053 | 70,0 |
| Boden 64 | 108,2 | 29153,0 | 0,00042 | 68,6 |
| Sammelboden 3 | 901,9 | 533739,0 | 0,00019 | 68,6 |
| Boden 65 | 225,5 | 533739,0 | 0,00005 | 68,6 |
| Boden 66 | 224,9 | 529658,7 | 0,00004 | 65,3 |
| Boden 67 | 224,2 | 525578,4 | 0,00003 | 61,9 |
| Boden 68 | 225,2 | 520550,7 | 0,00002 | 56,0 |
| Boden 69 | 226,1 | 515523,0 | 0,00001 | 50,1 |
| Boden 70 | 226,4 | 503046,4 | 0,00001 | 48,7 |
| Boden 71 | 204,0 | 204633,4 | 0,00003 | 47,4 |

(fortgesetzt)

| Volumenelement i | $m_{si}$ (kg) | $\dot{m}_i$ (kg/h) | $t_i^*$ (h) | $T_i$ (˚C) |
|---|---|---|---|---|
| Boden 72 | 204,5 | 204633,4 | 0,00002 | 44,8 |
| Boden 73 | 204,9 | 204633,4 | 0,00002 | 42,2 |
| Boden 74 | 206,0 | 201787,7 | 0,00001 | 37,9 |
| Boden 75 | 207,1 | 198942,0 | 0,00001 | 33,6 |

Damit ergibt sich ein $t_{ges}$ von nur noch 8,79 h.

Gleichzeitig werden bei diesem $t_{ges}$ als im trennwirksamen Raum abgetrennter (Meth)acrylmonomere enthaltender Stoffstrom mit dem höchsten Gewichtsanteil an Acrylsäure 90987 kg/h rohe Acrylsäure mit einem Acrylsäuregehalt von 96,9333 Gew.-% abgetrennt.

Eine Analyse ergibt, dass diese Verbesserung u.a. darauf zurückzuführen ist, dass die Flüssigphase im Sumpfraum 5 beim Beispiel infolge der verringerten Verweilzeit lediglich 19,27 Gew.-% Polyacrylsäure (Michael-Addukt) enthält.

**Patentansprüche**

**1.** Thermisches Trennverfahren zur Abtrennung wenigstens eines (Meth)acrylmonomere angereichert enthaltenden Stoffstroms aus einem (Meth)acrylmonomere enthaltenden Gemisch, umfassend den kontinuierlichen stationären Betrieb wenigstens einer thermischen Trennvorrichtung, die wenigstens einen trennwirksamen Raum mit oder ohne trennwirksame Einbauten umfaßt, in den wenigstens ein (Meth)acrylmonomere enthaltender Stoffstrom hinein - und aus dem wenigstens ein (Meth)acrylmonomere enthaltender Stoffstrom herausgeführt wird, wobei sich die durch den trennwirksamen Raum geführten individuellen chemischen Verbindungen, die Michael-Addition der (Meth)acrylmonomeren ausgenommen, beim Durchgang durch den trennwirksamen Raum, jeweils bezogen auf die insgesamt in den trennwirksamen Raum geführte Gesamtmenge der jeweiligen individuellen chemischen Verbindung, zu weniger als 20 mol-% chemisch verändern, mit der Maßgabe, dass

- der Stoffstrom, der insgesamt in den trennwirksamen Raum hineingeführt und gedanklich **dadurch** erzeugt wird, dass man die in den trennwirksamen Raum hineingeführten Einzelstoffströme addiert, X Gew.-% an von (Meth)acrylmonomeren verschiedenen Bestandteilen enthält,
- der Stoffstrom, der mit dem höchsten Gewichtsanteil an (Meth)acrylmonomeren aus dem trennwirksamen Raum herausgeführt wird, Y Gew.-% an von (Meth)acrylmonomeren verschiedenen Bestandteilen enthält,
- das Verhältnis X : Y ≥5 beträgt,
- der trennwirksame Raum, ausgenommen an den Stoffstromzufuhr- und an den Stoffstromausfuhrstellen, von einer festen Phase begrenzt wird und wenigstens einen Umlaufwärmetauscher umfasst, und
- das im trennwirksamen Raum mit Flüssigphase gefüllte Gesamtvolumen ≥1 m³ beträgt, wobei die Temperatur der Flüssigphase wenigstens teilweise ≥80˚C beträgt,

**dadurch gekennzeichnet,**

- **dass** bei einer Unterteilung des trennwirksamen Raums in n individuelle Volumenelemente, wobei sich die höchste und die niedrigste Temperatur der in einem einzelnen Volumenelement befindlichen Flüssigphase um nicht mehr als 2˚C unterscheiden und das Volumenelement ein im trennwirksamen Raum zusammenhängendes ist,

die Gesamtverweilzeit $t_{ges}$ mit

$$t_{ges} = \sum_{i=1}^{n} \frac{m_{si}}{\dot{m}_i} \cdot 2^A$$

≤ 20 h beträgt,

wobei

$A = (T_i - T_o)/10°C$,

$T_o = 100°C$,

$T_i$ = der arithmetische Mittelwert aus der in der Flüssigphase des Volumenelements i bestehenden höchsten und niedrigsten Temperatur in °C,

$m_{si}$ = die im Volumen der im Volumenelement i enthaltenen Flüssigphase insgesamt enthaltene Masse an (Meth)acrylmonomeren,

$\dot{m}_i$ = der aus dem Volumenelement i insgesamt herausgeführte Flüssigphasenmassenstrom, und

$$\sum_{i=1}^{n} =$$ die Summe über alle Volumenelemente i ist,

mit der Maßgabe, dass Volumenelemente i mit einer in ihnen enthaltenen Flüssigphasenmasse $m_i$ und $m_i/\dot{m}_i \geq 100$ h als Totraum - Volumenelemente ebenso wenig in die Summe über alle Volumenelemente i mit einbezogen werden wie Volumenelemente i, die keine Flüssigphase aufweisen und die Gesamtmenge der in den Totraum-Volumenelementen enthaltenen Flüssigphase nicht mehr als 5 Gew.-% der im trennwirksamen Raum insgesamt enthaltenen Flüssigphase beträgt.

2. Verfahren nach Anspruch 1, **dadurch gekennzeichnet, dass** X:Y $\geq 8$ beträgt.

3. Verfahren nach Anspruch 1 oder 2, **dadurch gekennzeichnet, dass** das im trennwirksamen Raum mit Flüssigphase gefüllte Gesamtvolumen $\geq 5$ m$^3$ beträgt.

4. Verfahren nach einem der Ansprüche 1 bis 3, **dadurch gekennzeichnet, dass** die Temperatur der Flüssigphase im trennwirksamen Raum wenigstens teilweise $\geq 100°C$ beträgt.

5. Verfahren nach einem der Ansprüche 1 bis 4, **dadurch gekennzeichnet, dass** das wenigstens eine (Meth)acrylmonomere ausgewählt ist aus der Gruppe umfassend Acrolein, Methacrolein, Acrylsäure, Methacrylsäure, Hydroxyethylacrylat, Hydroxyethylmethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, iso-Butylmethacrylat, n-Butylmethacrylat, tert.-Butylacrylat, tert.-Butylmethacrylat, Ethylacrylat, Ethylmethacrylat, 2-Ethylhexylacrylat, 2-Ethylhexylmethacrylat, N,N-Dimethylaminoethylmethacrylat und N,N-Dimethylaminoethylacrylat.

6. Verfahren nach einem der Ansprüche 1 bis 5, **dadurch gekennzeichnet, dass** $t_{ges} \leq 15$ h beträgt.

7. Verfahren nach einem der Ansprüche 1 bis 6, **dadurch gekennzeichnet, dass** $t_{ges} \leq 10$ h beträgt.

8. Verfahren nach einem der Ansprüche 1 bis 7, **dadurch gekennzeichnet, dass** der trennwirksame Raum eine trennwirksame Kolonne mit Stoffaustauschböden als trennwirksamen Einbauten umfasst.

9. Verfahren nach einem der Ansprüche 1 bis 8, **dadurch gekennzeichnet, dass** der Umlaufwärmetauscher ein Umlaufverdampfer ist.

10. Verfahren nach einem der Ansprüche 1 bis 9, **dadurch gekennzeichnet, dass** der Umlaufwärmetauscher ein Direktumlaufverdampfer ist.

11. Verfahren nach einem der Ansprüche 1 bis 10, **dadurch gekennzeichnet, dass** es sich bei dem thermischen Trennverfahren um die fraktionierende Kondensation des Produktgasgemisches einer heterogen katalysierten partiellen Gasphasenoxidation von Propen und/oder Propan zu Acrylsäure handelt.

12. Verfahren nach einem der Ansprüche 1 bis 11, **dadurch gekennzeichnet**, das $t_{ges} \leq 10$ h, das (Meth)acrylmonomere enthaltende Gemisch das Produktgasgemisch einer heterogen katalysierten partiellen Gasphasenoxidation von Propen und/oder Propan zu Acrylsäure mit einem Acrylsäuregehalt von 5 bis 15 Gew.-% und der Stoffstrom, der mit dem höchsten Gewichtsanteil an (Meth)acrylmonomeren aus dem trennwirksamen Raum herausgeführt wird, rohe Acrylsäure mit einem Acrylsäuregehalt $\geq 95$ Gew.-% ist.

13. Verfahren nach Anspruch 8, **dadurch gekennzeichnet, dass** es sich bei den Stoffaustauschböden der trennwirksamen Kolonne von unten nach oben zunächst um Dual-Flow-Böden, dann um hydraulisch abgedichtete Querströmböden und abschließend um Ventilböden handelt.

**Claims**

1. A thermal separating process for removing at least one stream comprising enriched (meth)acrylic monomers from a mixture comprising (meth)acrylic monomers, comprising the continuous steady-state operation of at least one thermal separating apparatus which comprises at least one separating space with or without separating internals, into which at least one stream comprising (meth)acrylic monomers is conducted and out of which at least one stream comprising (meth)acrylic monomers is conducted, where the individual chemical compounds conducted through the separating space, excluding the Michael addition of the (meth)acrylic monomers, as they pass through the separating space, based in each case on the total amount of the particular individual chemical compound conducted overall into the separating space, are chemically changed to an extent of less than 20 mol%, with the proviso that

- the stream which is conducted overall into the separating space and is obtained in a theoretical sense by adding the individual streams conducted into the separating space comprises X% by weight of constituents other than (meth)acrylic monomers,
- the stream which is conducted out of the separating space with the highest proportion by weight of (meth) acrylic monomers comprises Y% by weight of constituents other than (meth)acrylic monomers,
- the X : Y ratio is $\geq 5$,
- the separating space, except at the stream inlet and at the stream outlet points, is bounded by a solid phase and comprises at least one circulation heat exchanger, and
- the total volume filled with liquid phase in the separating space is $\geq 1$ m$^3$, and the temperature of the liquid phase, at least in places, is $\geq 80°C$,
wherein,
- in the case that the separating space is divided into n individual volume elements and the highest and the lowest temperatures of the liquid phase disposed in an individual volume element do not differ by more than 2°C and the volume element is continuous within the separating space,
the overall residence time $t_{ort}$,

$$t_{ort} = \sum_{i=1}^{n} \frac{m_{si}}{\dot{m}_i} \cdot 2^A$$

,

is $\leq 20$ h,
where

A= $(T_i-T_o)/10°C$,
$T_o = 100°C$,
$T_i$ = the arithmetic mean of the highest and lowest temperature existing in the liquid phase of the volume element i in °C,
$m_{si}$ = the total amount of (meth)acrylic monomers present in the volume of the liquid phase present in the volume element i,
$\dot{m}_i$ = the total amount of liquid phase stream conducted out of the volume element i, and

$$\sum_{i=1}^{n} = \text{ the sum over all volume elements i,}$$

with the proviso that the sum over all volume elements i includes neither volume elements i having a liquid phase mass $m_i$ present therein and $m_i/\dot{m}_i \geq 100$ h, as deadspace volume elements, nor volume elements i which have no liquid phase, and the total amount of the liquid phase present in the deadspace volume elements is not more than 5% by weight of the overall liquid phase present in the separating space.

**2.** The process according to claim 1, wherein X:Y is $\geq 8$.

**3.** The process according to either of claims 1 or 2, wherein the total volume filled with liquid phase in the separating space is $\geq 5$ m$^3$.

**4.** The process according to any of claims 1 to 3, wherein the temperature of the liquid phase in the separating space is, at least in places, $\geq 100°$C.

**5.** The process according to any of claims 1 to 4, wherein the at least one (meth)acrylic monomer is selected from the group consisting of acrolein, methacrolein, acrylic acid, methacrylic acid, hydroxyethyl acrylate, hydroxyethyl methacrylate, hydroxypropyl acrylate, hydroxypropyl methacrylate, glycidyl acrylate, glycidyl methacrylate, methyl acrylate, methyl methacrylate, n-butyl acrylate, isobutyl acrylate, isobutyl methacrylate, n-butyl methacrylate, tert-butyl acrylate, tert-butyl methacrylate, ethyl acrylate, ethyl methacrylate, 2-ethylhexyl acrylate, 2-ethylhexyl methacrylate, N,N-dimethylaminoethyl methacrylate and N,N-dimethylaminoethyl acrylate.

**6.** The process according to any of claims 1 to 5, wherein $t_{ort}$ is $\leq 15$ h.

**7.** The process according to any of claims 1 to 6, wherein $t_{ort}$ is $\leq 10$ h.

**8.** The process according to any of claims 1 to 7, wherein the separating space comprises a separating column having mass transfer trays as separating internals.

**9.** The process according to any of claims 1 to 8, wherein the circulation heat exchanger is a circulation evaporator.

**10.** The process according to any of claims 1 to 9, wherein the circulation heat exchanger is a direct circulation evaporator.

**11.** The process according to any of claims 1 to 10, wherein the thermal separating process is the fractional condensation of the product gas mixture of a heterogeneously catalyzed partial gas phase oxidation of propene and/or propane to acrylic acid.

**12.** The process according to any of claims 1 to 11, wherein $t_{ort}$ is $\leq 10$ h, the mixture comprising (meth)acrylic monomers is the product gas mixture of a heterogeneously catalyzed partial gas phase oxidation of propene and/or propane to acrylic acid and has an acrylic acid content of from 5 to 15% by weight, and the stream which is conducted out of the separating space with the highest proportion by weight of (meth)acrylic monomers is crude acrylic acid having an acrylic acid content of $\geq 95\%$ by weight.

**13.** The process according to claim 8, wherein the mass transfer trays of the separating column, from bottom to top, are initially dual-flow trays, then hydraulically sealed crossflow trays and finally valve trays.

**Revendications**

**1.** Procédé de séparation thermique pour la séparation d'au moins un courant enrichi en monomères (méth)acryliques à partir d'un mélange contenant des monomères (méth)acryliques, comprenant l'utilisation stationnaire continue d'au moins un dispositif de séparation thermique, qui comprend au moins une chambre de séparation avec ou sans constituants de séparation, dans laquelle est introduit au moins un courant contenant des monomères (méth) acryliques et de laquelle est déchargé au moins un courant contenant des monomères (méth)acryliques, les composés chimiques individuels qui traversent la chambre de séparation étant modifiés chimiquement à moins de 20 % en moles lors du passage par la chambre de séparation, à l'exception de l'addition de Michael des monomères (méth)acryliques, à chaque fois par rapport à la totalité de chaque composé chimique individuel introduit dans la chambre de séparation, à condition que

- le courant, qui est introduit en totalité dans la chambre de séparation et qui est théoriquement généré en ajoutant les courants individuels introduits dans la chambre de séparation, contienne X % en poids de constituants différents des monomères (méth)acryliques,
- le courant qui, avec la proportion en poids la plus élevée en monomères (méth)acryliques est déchargé de la chambre de séparation, contienne Y % en poids de constituants différents des monomères (méth)acryliques,
- le rapport X : Y $\geq 5$,

- la chambre de séparation, à l'exception des emplacements d'introduction du courant et de déchargement du courant, soit délimitée par une phase solide et comprenne au moins un échangeur de chaleur à circulation, et
- le volume total de phase liquide introduit dans la chambre de séparation soit $\geq 1$ m$^3$, la température de la phase liquide étant au moins partiellement a 80 °C,

**caractérisé en ce que**

- lors d'une division de la chambre de séparation en n éléments volumiques individuels, la température la plus haute et la température la plus basse d'une phase liquide se trouvant dans un élément volumique individuel ne différant pas de plus de 2 °C et l'élément volumique étant cohérent dans la chambre de séparation,

le temps de séjour total $t_{ges}$ avec

$$t_{ges} = \sum_{i=1}^{n} \frac{m_{si}}{\dot{m}_i} \times 2^A$$

est $\leq 20$ h,
avec
$A = (T_i - T_o) / 10$ °C
$T_o = 100$ °C,

$T_i$ = la moyenne arithmétique de la température la plus élevée et la plus basse dans la phase liquide de l'élément volumique i en °C,

$m_{si}$ = la masse totale de monomères (méth)acryliques contenue dans le volume de la phase liquide contenue dans l'élément volumique i,

$m_i$ = le débit massique total de phase liquide déchargé de l'élément volumique i, et

$$\sum_{i=1}^{n} = \text{la somme sur tous les éléments volumiques i,}$$

à condition que les éléments volumiques i contenant une masse de phase liquide $m_i$ et avec $m_1/m_i \geq 100$ h en tant qu'éléments volumique de volume mort ne soient pas davantage inclus dans la somme de tous les éléments volumiques i que les éléments volumiques i qui ne comportent pas de phase liquide, et que la totalité de la phase liquide présente dans les éléments volumiques du volume mort ne représente pas plus de 5 % en poids de la phase liquide présente au total dans la chambre de séparation.

2. Procédé selon la revendication 1, **caractérisé en ce que** X : Y $\geq 8$.

3. Procédé selon la revendication 1 ou 2, **caractérisé en ce que** le volume total de phase liquide introduit dans la chambre de séparation est $\geq 5$ m$^3$.

4. Procédé selon l'une quelconque des revendications 1 à 3, **caractérisé en ce que** la température de la phase liquide dans la chambre de séparation est au moins partiellement a 100 °C.

5. Procédé selon l'une quelconque des revendications 1 à 4, **caractérisé en ce que** le ou les monomères (méth)acryliques sont choisis dans le groupe comprenant l'acroléine, la méthacroléine, l'acide acrylique, l'acide méthacrylique, l'acrylate d'hydroxyéthyle, le méthacrylate d'hydroxyéthyle, l'acrylate d'hydroxypropyle, le méthacrylate d'hydroxypropyle, l'acrylate de glycidyle, le méthacrylate de glycidyle, l'acrylate de méthyle, le méthacrylate de méthyle, l'acrylate de n-butyle, l'acrylate d'iso-butyle, le méthacrylate d'iso-butyle, le méthacrylate de n-butyle, l'acrylate de tert.-butyle, le méthacrylate de tert.-butyle, l'acrylate d'éthyle, le méthacrylate d'éthyle, l'acrylate de 2-éthylhexyle, le méthacrylate de 2-éthylhexyle, le méthacrylate de N,N-diméthylaminoéthyle et l'acrylate de N,N-diméthylaminoéthyle.

6. Procédé selon l'une quelconque des revendications 1 à 5, **caractérisé en ce que** $t_{ges} \leq 15$ h.

7. Procédé selon l'une quelconque des revendications 1 à 6, **caractérisé en ce que** $t_{ges} \leq 10$ h.

8. Procédé selon l'une quelconque des revendications 1 à 7, **caractérisé en ce que** la chambre de séparation comporte une colonne de séparation munie de plateaux d'échange de substances en tant que constituants de séparation.

9. Procédé selon l'une quelconque des revendications 1 à 8, **caractérisé en ce que** l'échangeur thermique à circulation est un évaporateur à circulation.

10. Procédé selon l'une quelconque des revendications 1 à 9, **caractérisé en ce que** l'échangeur thermique à circulation est un évaporateur à circulation directe.

11. Procédé selon l'une quelconque des revendications 1 à 10, **caractérisé en ce que** le procédé de séparation thermique est la condensation fractionnée du mélange gazeux de produits d'une oxydation partielle en phase gazeuse sous catalyse hétérogène de propène et/ou de propane en acide acrylique.

12. Procédé selon l'une quelconque des revendications 1 à 11, **caractérisé en ce que** $t_{ges} \leq 10$ h, **en ce que** le mélange contenant des monomères (méth)acryliques est le mélange gazeux de produits d'une oxydation partielle en phase gazeuse sous catalyse hétérogène de propène et/ou de propane en acide acrylique ayant une teneur en acide acrylique de 5 à 15 % en poids et le courant qui, avec la proportion en poids la plus élevée en monomères (méth)acryliques, est déchargé de la chambre de séparation, est de l'acide acrylique brut ayant une teneur en acide acrylique $\geq 95$ % en poids.

13. Procédé selon la revendication 8, **caractérisé en ce que** les plateaux d'échange de substances de la colonne de séparation sont, de bas en haut, tout d'abord des plateaux à double courant, puis des plateaux à courant transversal scellés hydrauliquement, et enfin des plateaux à clapets.

EP 1 646 603 B1

# FIG.1

32

# FIG.2

# FIG.3

# FIG.4

# FIG.5

# FIG.6

# FIG.7

FIG.8

FIG.9

FIG.10

FIG.11

# FIG.12

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 19924532 A
- EP 717019 A
- EP 1125912 A
- EP 982289 A
- EP 982287 A
- DE 19606877 A
- DE 1011527 A
- DE 10224341 A
- DE 10218419 A
- DE 10247240 A
- DE 10243625 A
- EP 733617 A
- EP 765861 A
- DE 19536191 A
- DE 19851984 A
- DE 19927722 A
- EP 780360 A
- EP 780359 A
- WO 9808798 A
- WO 9748669 A
- DE 19924533 A
- EP 648732 A
- EP 270999 A
- EP 717029 A
- DE 4308087 A
- EP 990636 A
- EP 861820 A
- EP 778255 A
- EP 551111 A
- EP 695736 A
- EP 1026145 A
- DE 10251328 A
- DE 10159825 A
- WO 0250011 A
- WO 03047714 A
- DE 10156016 A
- DE 10223058 A
- DE 10036880 A
- EP 616998 A

### In der Beschreibung aufgeführte Nicht-Patentliteratur

- **Johann Stichlmair.** Grundlagen der Dimensionierung des Gas/Flüssigkeit-Kontaktapparates. Verlag Chemie, 1978
- Grundlagen der Dimensionierung von Kolonnenböden. **von Dr.-Ing. ; Klaus Hoppe ; Dr.-Ing.** Technische Fortschrittsberichte. Verlag Theodor Steinkopff, 1967, vol. 61
- **Klaus Hoppe ; Dr.-Ing.** Manfred Mittelstrass. Verlag Theodor Steinkopff